# EUROPEAN PATENT APPLICATION

(11) **EP 2 959 826 A2**
(43) Date of publication of application: **30.12.2015**
(21) Application number: 15165383.9
(22) Date of filing: 28.04.2015
(51) Int. Cl.: A61B 5/00, A61B 8/00

(54) **ULTRASONIC DIAGNOSIS APPARATUS AND BIOMEDICAL LIGHT MEASURING APPARATUS**

(30) Priority: 28.04.2014 JP 2014093033; 19.09.2014 JP 2014191961
(71) Applicant: Kabushiki Kaisha Toshiba, Tokyo 105-8001 (JP)
(72) Inventor: Urano, Taeko, Tokyo 105-8001 (JP); Takayama, Satoshi, Tokyo 105-8001 (JP); Nakanishi, Tsutomu, Tokyo 105-8001 (JP); Nakamura, Kenji, Tokyo 105-8001 (JP)
(74) Representative: Hoffmann Eitle

(57) **Abstract**

In general, according to one embodiment, an ultrasonic diagnostic apparatus is characterized by comprising a plurality of light emission units, a control unit, a plurality of light detection units and a calculation unit. The light emission units are arranged around an ultrasonic transmitting and receiving surface of an ultrasonic probe included by the ultrasonic diagnostic apparatus and emit light including a component of a specific wavelength to the subject, the particular wavelength being included in an absorption wavelength range of a body. The control unit drives a plurality of the light emission units at different frequencies. The plurality of light detection units are arranged around the ultrasonic transmitting and receiving surface and detect the light of the specified wavelength reflected inside the subject. The calculation unit calculates intensity of the light of the specified wavelength at different frequencies.

## Description

### FIELD

Embodiments described herein relate generally to an ultrasonic diagnosis apparatus and a biomedical light measuring apparatus.

### BACKGROUND

Various techniques of technology to noninvasively measure the inside of a living body are known. Optical measurement as one of such techniques has advantages of no exposure to radiation and being able to select a compound to be measured by selecting the wavelength. A general biomedical light measuring apparatus calculates various kinds of biomedical information based on measurements of light, with which the inside of the living body is irradiated transdermally by a light irradiation unit being pressed against the skin surface of the living body, emitted out of the body by passing through the skin again after being transmitted or reflected inside the body. The foundation on which to determine the presence of an abnormal tissue inside the body by the optical measurement is a difference in absorption coefficient of light from a normal tissue. That is, the absorption coefficient of an abnormal tissue inside the body is different and thus, a difference of the amount of detected light in accordance with a difference of the amount of absorption arises. In other words, if an inverse problem is solved from the amount of detected light, the absorption coefficient of the abnormal tissue can be determined and properties of the abnormal tissue can be judged from the determined absorption coefficient. In addition, the measured position of the abnormal tissue and depth are analyzed from the measured light. The analysis techniques include a technique of adjusting the distance between a light irradiation unit (hereinafter, abbreviated as a light source) and a detector (space resolution method), a technique of obtaining depth information from a difference of times when lights arrive by using a light source whose intensity changes with time (time resolution method), and further a method of combining these techniques. By these analysis methods, a biomedical light measuring apparatus capable of acquiring a high-quality signal can be realized. However, a problem of low spatial resolution is posed for visualization of information by light inside a body. Further, it is necessary to operate a large amount of data using a complex algorithm to obtain correct position information from a detected result of reflected light, which is not to be determined in real time.

Application whose implementability is considered to be high in biomedical light measurement includes breast cancer examination (see Patent Literature 1). However, the aforementioned optical measurement alone has problems of the space resolution and analysis time and thus, it is desirable to improve examination performance by combining with another modality. Thus, we propose a method of supplementing low spatial resolution of light by using morphologic information of an ultrasonic echo (see Patent Literature 2). While morphologic features in tissues and a component distribution of a morphologic feature portion are expected to be determined in a shorter time than in the past by this method, there is room for improvement of an immediate determination.

Incidentally, breast cancer is one of main causes of death of women. Screening and early diagnosis of breast cancer are very valuable to decrease the mortality and to curb health care costs. In the current practice, breast tissues are palpated and mammography to search for any suspicious tissue deformity is carried out. If a suspicious site is detected in the mammogram, ultrasonic imaging is carried out and further, surgical tissue examination is carried out. A series of these examinations needs quite a long time to reach a final conclusion. There is also a problem of difficulty of producing sensitivity in mammography for the younger generation before the menopause with many mammary glands. Therefore, the meaning of screening by ultrasonic imaging is significant particularly for the younger generation.

In the ultrasonic imaging in general, ultrasonic still images by a qualified operator are collected and a determination is made by a specialist X-ray reader (or X-ray readers) according to morphologic information on the image. In view of the danger of oversight due to fatigue or insufficient concentration of the operator in the diagnosis, screening by one operator is limited to maximally 50 persons per day.

Knowledge and experience of the operator are very important to collect still images capturing morphologic features when ultrasonic images are taken. In addition, a degree of skill is needed for accurate and quick screening. For example, the standard examination time per subject is 5 to 10 min, but depending on the skill level of the operator, a still longer time may be needed. That is, in the screening by the current ultrasonic imaging, appropriateness of image collection may vary depending on proficiency of the operator. Further, in addition to the need to constantly watch images, the judgment is left to the operator alone when images are collected and thus, even if the operator is proficient, the psychological burden is heavy. A method of collecting all image information by viewing dynamic images is known, but in such a case, the burden on the X-ray reader is heavy.

To solve such a problem of ultrasonic image diagnosis, a method of lessening the burden of an operator by guiding the measuring position of an ultrasonic probe in a plane direction based on metabolism information of the body obtained by biomedical light measurement is proposed (see Patent Literature 3). According to such a method, an abnormal site can be detected and judged in a shorter time than in the past and relatively easily in ultrasonic imaging.

However, there is still room for improvement of the conventional biomedical light measuring method, the biomedical light measuring apparatus using the technique, and ultrasonic probe position guidance of ultrasonic imaging from the viewpoint of, for example, reducing the amount of data to be calculated.

### BRIEF DESCRIPTION OF THE DRAWING

FIG. 1 is a block diagram of a biomedical light measuring apparatus 4 including a light probe 40 and an optical measurement processing unit 42;
FIG. 2 is a block diagram of an ultrasonic diagnosis apparatus 1 into which the biomedical light measuring apparatus 4 according to the present embodiment is incorporated;
FIG. 3 is a diagram illustrating a contact surface PS with a body of an interface unit P;
FIG. 4 is a diagram illustrating an influence on light intensity of a pressing force of the interface unit P on the body;
FIG. 5 is a diagram illustrating the contact surface PS with the body of the interface unit P;
FIG. 6 is a diagram showing an arrangement example of a light irradiation unit 400 and a light detection unit 401 on the surface of a subject;
FIGS. 7A and 7B are diagrams showing results of simulation by the Monte Carlo method of paths inside the body of light reaching the light detection unit 401;
FIGS. 8A and 8B are diagrams showing results of simulation by the Monte Carlo method of paths inside the body of light reaching the light detection unit 401;
FIG. 9 is a diagram showing the contact surface PS with the body of the interface unit P and a diagram illustrating a detection signal discrimination function;
FIG. 10 is a diagram showing the contact surface PS with the body of the interface unit P and a diagram illustrating the detection signal discrimination function;
FIG. 11 is a diagram showing the contact surface PS with the body of the interface unit P and a diagram illustrating the detection signal discrimination function;
FIG. 12 shows a flow chart of guidance processing of an ultrasonic probe using the ultrasonic diagnosis apparatus 1;
FIG. 13 is a block diagram of a biomedical light measuring apparatus 4 including a light probe 40 and an optical measurement processing unit 42;
FIG. 14 is a diagram when a probe P is viewed from a subject contact surface side and is a diagram showing an arrangement example of a light irradiation unit 400 and a light detection unit 401 with respect to an ultrasonic transmitting and receiving surface of an ultrasonic probe 12;
FIG. 15 is a diagram showing an example of the configuration of an optical measuring system of the biomedical light measuring apparatus 4;
FIG. 16 is a diagram showing Modification 1 of the configuration of the optical measuring system of the biomedical light measuring apparatus 4;
FIG. 17 is a diagram showing Modification 2 of the configuration of the optical measuring system of the biomedical light measuring apparatus 4;
FIGS. 18A and 18B are diagrams illustrating an ultrasonic probe guiding function held by the ultrasonic diagnosis apparatus;
FIG. 19A is a graph showing a correlation of light intensity between the wavelength (about 765 nm) at which deoxygenated hemoglobin is detected and a reference wavelength (about 809 nm) when measured in the same light source position and the same detection position. FIG. 19B is a graph showing the correlation of light intensity between the wavelength (about 855 nm) at which oxygenated hemoglobin is detected and the reference wavelength (about 809 nm) when similarly measured in the same light source position and the same detection position;
FIG. 20A is a graph showing changes with time of voltages output by each of the detection units 401 (ch1 = 15 mm, ch3 = 25 mm, ch5 = 35 mm) having different distances from the light source by an operation of pressing and releasing the probe P on the surface of the body. FIG. 20B is a graph showing a correlation of detected light intensity between channels normalized by intensity of the shortest distance ch1 = 15 mm;
FIGS. 21A and 21B show an example of a calibration sequence that calibrates output variations in light intensity generated in biomedical light measurements based on the reference wavelength;
FIG. 22 is a block diagram of the biomedical light measuring apparatus 4 to which a reference wavelength light intensity calibration function is added;
FIG. 23 is an example of the configuration of the optical measuring system of the biomedical light measuring apparatus 4 to which the reference wavelength light intensity calibration is added;
FIG. 24 is an example of the configuration of the optical measuring system of the biomedical light measuring apparatus 4 to which the reference wavelength light intensity calibration and a precise measuring function of oxygen saturation is added;
FIG. 25 is another example of the configuration of the optical measuring system of the biomedical light measuring apparatus 4 to which the reference wavelength light intensity calibration and the precise measuring function of oxygen saturation is added; and
FIG. 26 is another example of the configuration of the optical measuring system of the biomedical light measuring apparatus 4 to which the reference wavelength light intensity calibration is added.
FIG. 27 is a diagram when an interface unit P according to a third embodiment is viewed from the subject contact surface side.

### DETAILED DESCRIPTION

In general, according to one embodiment, an ultrasonic probe, an image generation unit, an ultrasonic diagnostic apparatus comprises a light emission unit, a control unit, a control unit, a plurality of light detection units and a calculation unit. The ultrasonic probe transmits an ultrasonic wave to a subject and receives the ultrasonic wave reflected inside the subject, via the ultrasonic transmitting and receiving surface. The image generation unit generates an ultrasonic image using the ultrasonic wave received by the ultrasonic probe. The light emission unit is arranged around an ultrasonic transmitting and receiving surface of an ultrasonic probe included by the ultrasonic diagnostic apparatus and emit light including a component of a specific wavelength to the subject, the particular wavelength being included in an absorption wavelength range of a body. The control unit drives a plurality of the light emission units at different frequencies. The plurality of light detection units are arranged around the ultrasonic transmitting and receiving surface and detect the light of the specified wavelength reflected inside the subject. The calculation unit calculates intensity of the light of the specified wavelength at different frequencies.

### (First embodiment)

Hereinafter, a biomedical light measuring apparatus and an ultrasonic diagnosis apparatus according to the present embodiment will be described with reference to the drawings. In the description that follows, the same reference numerals are attached to structural elements having substantially the same function and configuration and a duplicate description will be provided only if necessary.

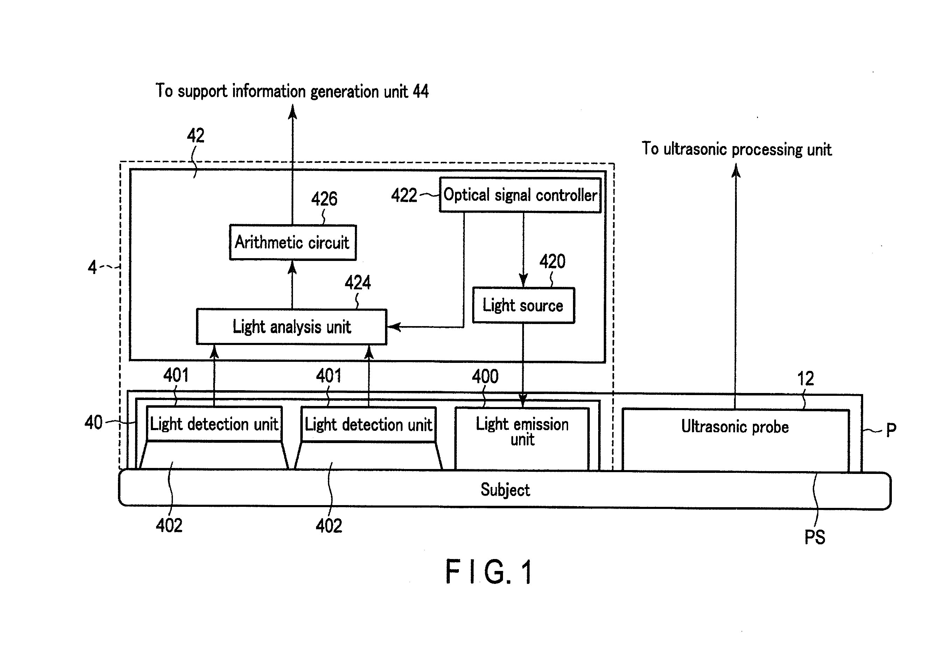

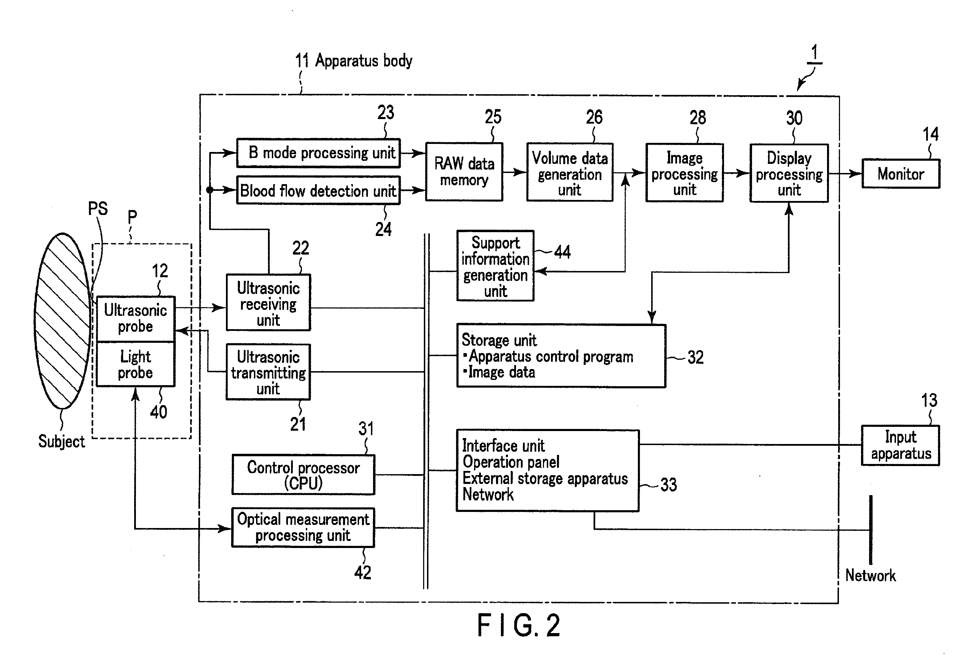

FIG. 1 is a block diagram of a biomedical light measuring apparatus 4 including a light probe 40 and an optical measurement processing unit 42. FIG. 2 is a block diagram of an ultrasonic diagnosis apparatus 1 into which the biomedical light measuring apparatus 4 according to the present embodiment is incorporated.

The biomedical light measuring apparatus 4 includes the light probe 40 and the optical measurement processing unit 42 and also a support information generation unit 44 that generates support information to support an arrangement operation of an ultrasonic probe 12. In the present embodiment, as shown in FIG. 2, the biomedical light measuring apparatus 4 incorporated into the ultrasonic diagnosis apparatus 1 (structured integrally with the ultrasonic diagnosis apparatus 1) will be described. However, the present embodiment is not limited to such an example and the biomedical light measuring apparatus and the ultrasonic diagnosis apparatus may be separately structured.

The light probe 40 includes at least one light irradiation unit 400, a plurality of light detection units 401, and a light guiding unit 402 to improve light detection efficiency. The light irradiation unit 400 irradiates a subject with light (near infrared light) generated by a light source 420 substantially vertically toward the subject. The light detection unit 401 has a contact surface formed from, for example, an end of an optical fiber and includes a plurality of detection elements that make a photoelectric conversion of reflected light from inside the subject input from the contact surface via a light wave-guiding unit. As the detection element, for example, in addition to a light receiving element such as a photo-diode and a photo-transistor, CCD, APD, or a photomultiplier can be adopted. A optical matching layer may be provided on the contact surface of the light irradiation unit 400 with the subject.

The light guiding unit 402 is provided, as shown in FIG. 1, between each of the light detection units 401 and the subject surface and has a structure in a conical shape (for example a tapering structure) in which an opening area on the subject surface side is larger than that on a detection surface side of the light detection unit 401. The light guiding unit 402 guides (or condenses) light from the subject based on irradiation light from the light irradiation unit 400 to dramatically improve light detection efficiency by each of the light detection units 401. The opening area of the light guiding unit 402 depends on the angle of aperture in a tapering shape of the light guiding unit 402. Incidentally, an optical fiber may be provided between the detection surface of the light detection unit 401 and the light guiding unit 402. In such a case, the opening area on the light guiding unit side of the optical fiber is formed so as to be smaller than the opening area on the surface side of the subject of the light guiding unit.

To evaluate the presence/absence of an abnormal tissue portion and properties thereof in biomedical light measurement, it is necessary to compare intensity of emitted light when there is an abnormal tissue portion and when there is no abnormal tissue portion. Further, when used for ultrasonic image diagnosis, it is necessary to detect an approaching state of the position to be used for ultrasonic imaging and notify the operator of the approaching state and to guide the ultrasonic probe (and the light probe 40), it is necessary for light intensity detected by following scanning of the light probe 40 to have an SN ratio capable of undergoing a computation. In the biomedical light measuring apparatus according to the present embodiment, as will be described later, the SN ratio can dramatically be improved compared with the past by the light guiding unit 402.

The optical measurement processing unit 42 includes the light source 420, an optical signal controller 422, a light analysis unit 424, and an arithmetic circuit 426. The light source 420 is a light-emitting element such as a semiconductor laser, a light-emitting diode, a solid-state laser, or a gas laser that generates light of a wavelength whose absorption in a body is small (for example, light in the range of 600 nm to 1000 nm as a wavelength range called an in vivo window) or light of a specific wavelength whose amount of absorption increases in an abnormal site (for example, light in the wavelength range called the in vivo window and also in the wavelength range of 750 to 860 nm absorbed by hemoglobin in blood). Light generated by the light source 420 is supplied to the light irradiation unit 400 via the light wave-guiding unit including the optical fiber and a thin light wave-guiding unit (or directly via propagation through the space). Incidentally, the light source 420 may be configured integrally with the light irradiation unit 400.

The optical signal controller 422 controls the biomedical light measuring apparatus 4 dynamically or statically. For example, under the control of a control processor 31 of the ultrasonic diagnosis apparatus 1, the optical signal controller 422 controls the light source 420 such that light is irradiated from the light irradiation unit 400 in the predetermined interval, frequency, intensity, and intensity variation period T. The optical signal controller 422 also controls the light analysis unit 424 such that light analysis processing is performed in the predetermined interval.

The light analysis unit 424 discriminates optical signals detected by a plurality of the detection units 401 according to a detection signal discrimination function described later. The light analysis unit 424 amplifies an analog signal input from the light detection unit 401 and then converts the analog signal into a digital signal. Further, the light analysis unit 424 analyzes changes in intensity of detection signals between the light detection units 401.

The arithmetic circuit 426 calculates changes in intensity of light between the light detection units 401 using the discriminated optical signals and solves an inverse problem consistently to calculate the depth of an abnormal site showing a predetermined optical absorption coefficient (for example, a site that absorbs more light of a specific wavelength than a normal tissue) inside the subject from the surface of the subject and also a three-dimensional position and a distance of the abnormal site relative to a predetermined position (for example, the light irradiation unit 400 or the center of an ultrasonic transmitting and receiving surface 12). Incidentally, the arithmetic circuit 426 may also calculate the degree of contact between the ultrasonic probe 12 and the surface of the subject based on changes in intensity of detection light between the light detection units 401. Calculation results by the arithmetic circuit 426 are sent out to the support information generation unit 44.

The support information generation unit 44 generates support information that instructs or guides the position, posture, and orientation of the ultrasonic probe 12 based on determination results and calculation results of the arithmetic circuit 426 to bring the transmitting and receiving surface of the ultrasonic probe 12 into close contact with the subject.

Next, the ultrasonic diagnosis apparatus 1 will be described. The ultrasonic diagnosis apparatus 1 includes, as shown in FIG. 2, a portion related to the biomedical light measuring apparatus 4 described above and a portion related to ultrasonic imaging. The portion related to ultrasonic imaging includes the ultrasonic probe 12, an input apparatus 13, a monitor 14, an ultrasonic transmitting unit 21, an ultrasonic receiving unit 22, a B mode processing unit 23, a blood flow detection unit 24, a RAW data memory 25, a volume data generation unit 26, an image processing unit 28, a display processing unit 30, the control processor (CPU) 31, a storage unit 32, and an external interface unit 33. The light probe 40 and the ultrasonic probe 12 of the biomedical light measuring apparatus 4 constitute an interface unit P.

The ultrasonic probe 12 is a device (probe) that transmits an ultrasonic wave to a subject, typically a body, and receives a reflected wave from the subject based on the transmitted ultrasonic wave and has a plurality of piezoelectric transducers, matching layers, and backing materials arranged at the tip thereof. A light irradiation surface of the light irradiation unit 400 and a light detection surface of the plurality of light detection units 401 are arranged, as will be described later, in a predetermined form near the ultrasonic transmitting and receiving surface of the ultrasonic probe 12. The input apparatus 13 is connected to an apparatus body 11 and includes various switches, buttons, a trackball, a mouse, a keyboard or the like to incorporate various instructions to operate the ultrasonic diagnosis apparatus 1 and the biomedical light measuring apparatus. The monitor 14 displays morphological information inside a body, blood flow information, and support information generated by the support information generation unit 44 in a predetermined form.

The interface unit P is an interface that arranges and fixes the ultrasonic probe 12 and the light probe 40 in a predetermined physical relationship and brings the light irradiation surface, light detection surface, and ultrasonic transmitting and receiving surface into close contact with a body. The configuration of the interface unit P will be described in detail later.

The ultrasonic transmitting unit 21 generates a trigger pulse to form a transmitting ultrasonic wave at a predetermined rate frequency fr Hz (period: 1/fr s) and applies a drive pulse to the probe 12 after giving a predetermined delay time. The ultrasonic receiving unit 22 amplifies an echo signal captured via the probe 12 in each channel and makes and A/D conversion of the signal and then performs addition processing to give a necessary delay time. The B mode processing unit 23 receives an echo signal from the ultrasonic receiving unit 22 and performs logarithmic amplification, envelope detection processing and the like thereon to generate data in which signal strength is represented by brightness in luminance. The blood flow detection unit 24 extracts a blood flow signal from an echo signal received from the receiving unit 22 to generate blood flow data (blood flow information such as the average speed, dispersion, and power).

The RAW data memory 25 generates RAW data by using a plurality of pieces of B mode data received from the B mode processing unit 23 and the blood flow detection unit 24. The volume data generation unit 26 generates B mode volume data and blood flow volume data by making a RAW-voxel conversion including interpolation processing to which spatial position information is added. The image processing unit 28 performs predetermined image processing such as volume rendering, multi planar reconstruction (MPR), and maximum intensity projection (MIP) on volume data received from the volume data generation unit 26. The display processing unit 30 performs various kinds of processing of the dynamic range, luminance (brightness), contrast, y curve correction, RGB conversion and the like on various kinds of image data generated or processed by the image processing unit 28. The control processor 31 has a function as an information processing apparatus (computer) and controls the operation of each structural element. The storage unit 32 stores dedicated programs, imaged volume data, diagnosis information (the patient ID, doctor's findings and the like), diagnostic protocol, transmission and reception conditions, captured images, and other data groups.

The external interface unit 33 is an interface for the input apparatus 13, a network, and a new external storage apparatus (not shown). An external biomedical light measuring apparatus can also be connected to the ultrasonic diagnosis apparatus body 11 via the external interface unit 33. Data such as ultrasonic images obtained by the apparatus and analysis results can be transferred to another apparatus by the external interface unit 33 via a network.

### (Interface unit)

First, the arrangement (or the relative physical relationship) of the ultrasonic transmitting and receiving surface of the ultrasonic probe 12 and the light irradiation surface and the light detection surface of the light probe 40 in the interface unit P will be described.

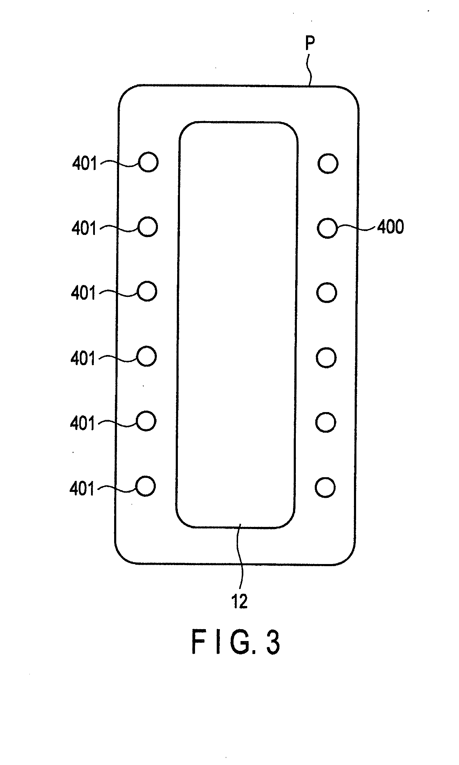

FIG. 3 is a diagram illustrating a contact surface PS with a body of the interface unit P. As shown in FIG. 3, the ultrasonic transmitting and receiving surface of the ultrasonic probe 12 is arranged in the center portion of the contact surface PS of the interface unit P and the light irradiation surface and the light detection surface of the light probe 40 are arranged in a region sandwiched between two straight lines obtained by extending short sides of the ultrasonic transmitting and receiving surface. The light irradiation surface and the light detection surface are arranged with respect to the ultrasonic transmitting and receiving surface as described above in consideration of an influence of the interface unit P on the body by close contact (pressing force). A detailed description thereof will be provided below.

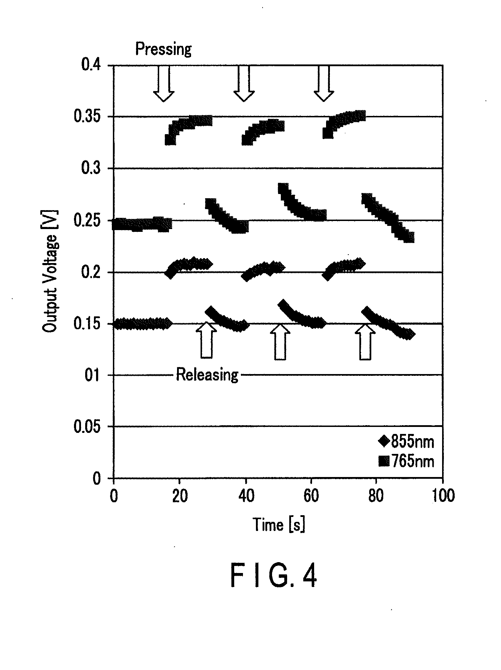

FIG. 4 is a graph obtained by irradiating the inside of the abdomen of the body from the surface with lights of two wavelengths 855 nm and 765 nm while pressing the interface unit P thereon and plotting obtained light intensity and a diagram illustrating the influence of the interface unit P on the body by a pressing force. As is evident from FIG. 4, the measured light intensity rises when the interface unit P is pressed and the light intensity attenuates when pressing of the interface unit P is released. This is because blood is pressed out by the surface being pressed by the interface unit P and the amount of light absorbed by blood is decreased with decreased blood and as a result, the amount of received light is considered to increase. When ultrasonic imaging is performed by pressing the ultrasonic probe on the body surface, such a phenomenon can also be estimated from a report confirming a phenomenon in which the subcutaneous tissue is crushed in the body from an ultrasonic image (Non-Patent Literature: 2012 IEEE International Conference on Intelligent Robots and Systems 1284-91 2012) and a report on a phenomenon in which light intensity is changed by pressing a probe in subcutaneous fat thickness measuring technology (Non-Patent Literature: Panasonic Electric Works Technical Report 58, 40-44 2010).

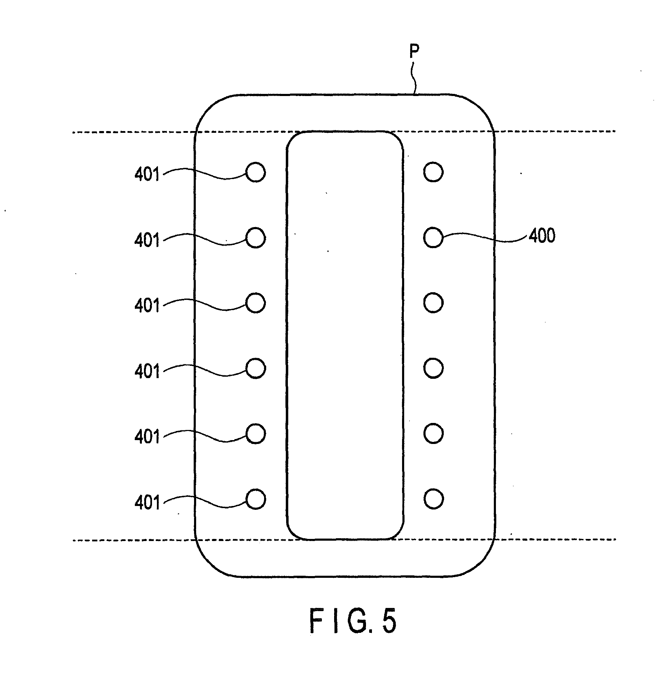

In view of the above consideration results, the present inventors found that it is important to match a pressed state when an ultrasonic image is measured and a pressed state when an optical signal is measured while the interface unit P is in close contact with the body. Results of continued studies show that it is necessary to make optical measurements in a range sandwiched between two straight lines obtained by extending short sides of the ultrasonic transmitting and receiving surface in a substantially rectangular shape (including a shape that can practically be considered to be rectangular, though corners do not form a right angle) of the ultrasonic probe 12. Therefore, the interface unit P of the ultrasonic diagnosis apparatus 1 adopts the configuration in which, as shown in FIG. 5, the irradiation surface of the light irradiation unit 400 and the detection surface of each light detection unit are arranged in a region sandwiched between two straight lines obtained by extending short sides of the ultrasonic probe 12.

Next, optical measurement conditions and the configuration of the interface unit P to improve operability will be described. Important data to reconfigure a light absorption image in substantially the same position as the position of an ultrasonic scanning section using the ultrasonic probe 12 is measured from around the ultrasonic transmitting and receiving surface of the ultrasonic probe 12. If the fact that, as described above, the subcutaneous tissue is crushed by a pressing force of the interface unit P or the ultrasonic probe 12, it is preferable to be able to control the pressing force of the contact surface PS of the interface unit P including the light irradiation surface and the light detection surface on the body surface at a constant level to perform an operation by combining measured data measured in different positions of the ultrasonic probe 12.

A publicly known example (Non-Patent Literature: 35^{th} Annual International Conference of the IEEE EMBS 140-143 2013) discloses an ultrasonic probe capable of measuring a pressing force and shows that variations of the pressing force during scanning of the ultrasonic probe are large. An ultrasonic probe whose pressing force is controlled is also disclosed by Non-Patent Literature: 2012 IEEE International Conference on Intelligent Robots and Systems 1284-91 2012. However, the configuration to control the pressing force of an ultrasonic probe used commonly has not yet been proposed.

In general, a skilled clinical operator is said to scan an ultrasonic probe with a small constant pressing force. In reality, however, the pressing force varies depending on the patient undergoing an examination and the site. The interface unit P is desirably a unit capable of adjusting the pressing force depending on also such individual differences of an operator or an examinee.

In view of the above considerations, the present inventors adopt, as a result of studying probes of various shapes, the configuration in which, as shown in FIGS. 3 and 5, the light irradiation surface of the light irradiation unit 400 and the light detection surface of each of the plurality of light detection units 401 form a substantially straight line with respect to the long side of the ultrasonic transmitting and receiving surface. By adopting such a configuration, optical data can be measured in a position close to the ultrasonic transmitting and receiving surface of the ultrasonic probe 12 and also the image can be reconfigured by using optical data acquired substantially simultaneously (that is, substantially under the same physical conditions as those of ultrasonic scanning even if the interface unit P is pressed against the body) with ultrasonic scanning. The shape of the interface unit P can be made as similar to the shape of the ultrasonic probe 12 as possible and also operability equal to that of a conventional ultrasonic probe can be realized.

Next, the configuration to guarantee close contact between the contact surface PS of the interface unit P and the body surface will be described. In general, the index of refraction of a body is about 1.4 and when light enters the body, if the air (whose index of refraction is 1) is present between the light irradiation unit and the body, a loss of light by reflection arises. Also when light is fetched from inside the body, the presence of the air causes a signal to attenuate.

To reduce such losses, the interface unit P adopts the configuration in which the ultrasonic transmitting and receiving surface, the light irradiation surface, and the light detection surface are contained in the contact surface PS and also the contact surface PS is a gentle plane. By adopting such a configuration, when the interface unit P is pressed against the body, the contact surface PS can be brought into close contact with the body surface with a substantially equal pressure. In addition, by adopting a gentle plane for the contact surface PS, when a matching agent (for example, an ultrasonic jelly) is applied, the air can efficiently be removed.

When a photo-diode or photo-transistor is used as the light detection unit 401, the applied voltage is low and thus, the light detection unit 401 installed inside the interface unit P can safely be brought into contact with the body. On the other hand, when APD or a photomultiplier is used as the light detection unit 401, these high-sensitivity devices are elements that apply a high voltage and thus, there is a danger of an electric shock. In such a case, an optical fiber is provided on the detection surface of the light detection unit 401 to bring the optical fiber into contact with the body while the contact surface PS being guaranteed to be a gentle plane so that light guided by the optical fiber is desirably received without be wasted.

### (Detection signal discrimination function)

Next, the detection signal discrimination function held by the biomedical light measuring apparatus 4 will be described. This function determines the range to be calculated using each distance between the light irradiation position into the body and each detection position of emitted light from inside the body as a reference and discriminates an optical signal used for calculation from a plurality of detected optical signals.

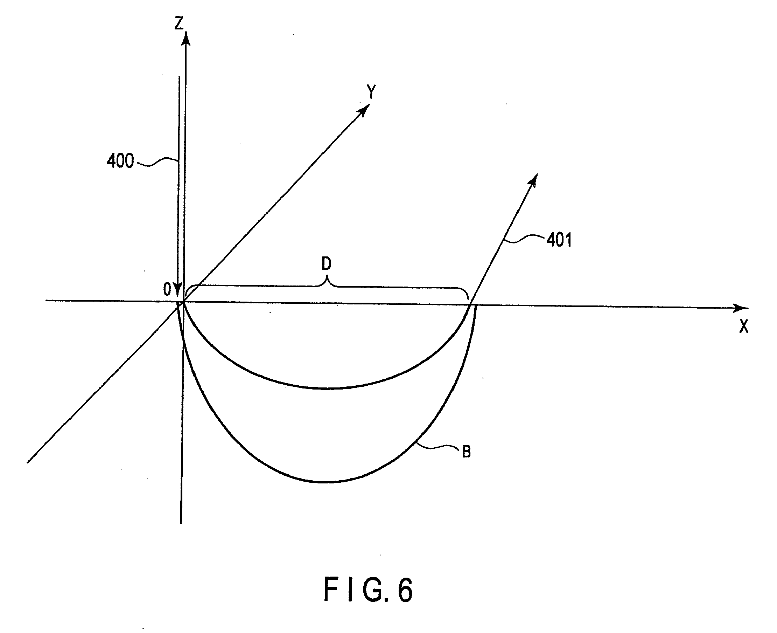

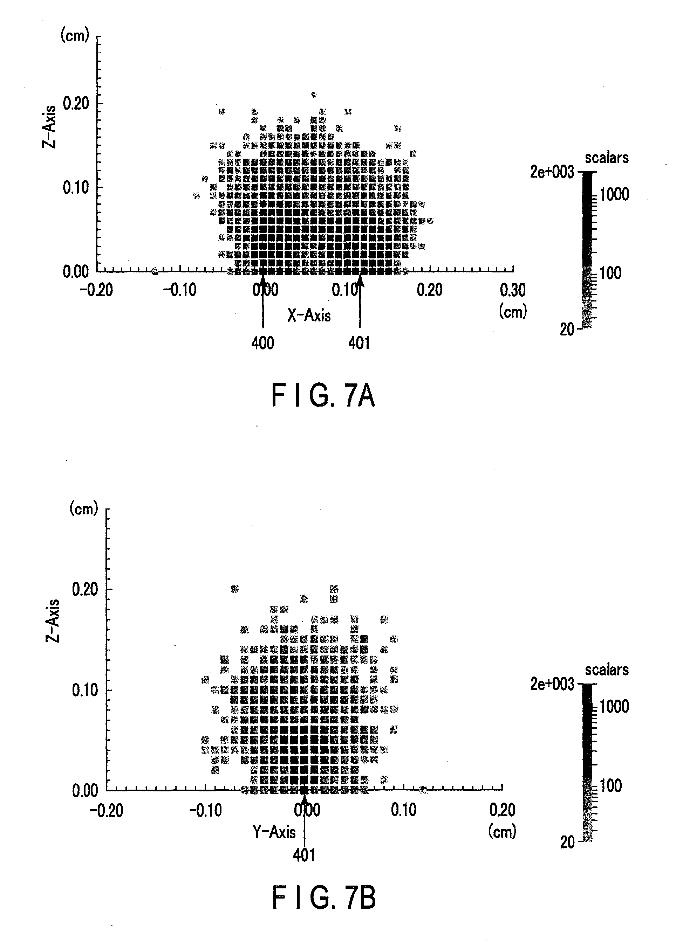

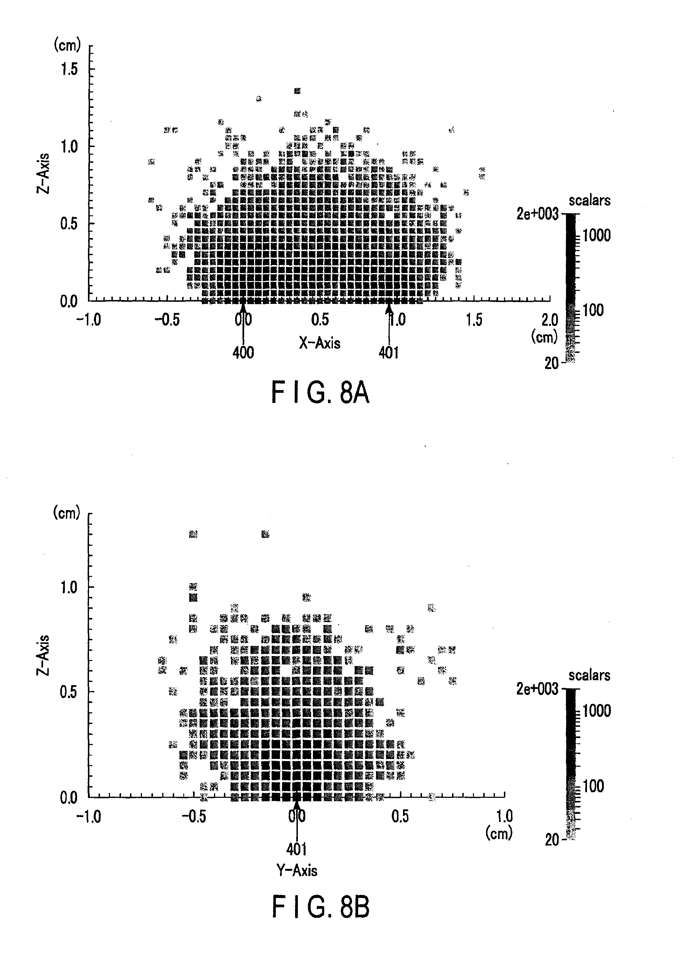

First, the theoretical background will be described. FIG. 6 is a diagram showing an arrangement example of the light irradiation unit 400 and the light detection unit 401 on the surface of a subject. The light irradiated from the light irradiation unit 400 spreads gradually as is called a banana shape and is reflected (scattered) repeatedly inside the body and then emitted out of the body again before being detected by the detection unit 401. FIGS. 7A to 8B are diagrams showing results of simulation by the Monte Carlo method of paths of light inside the body reaching the light detection unit 401. That is, FIG. 7A is a simulation result of light propagation paths inside the body in the Z-X plane when D (distance between the light irradiation unit 400 and the light detection unit 401) = 1 mm and FIG. 7B is a simulation result of light propagation paths inside the body in the Z-Y plane when D = 1 mm. FIG. 8A is a simulation result of light propagation paths inside the body in the Z-X plane when D = 10 mm and FIG. 8B is a simulation result of light propagation paths inside the body in the Z-Y plane when D = 10 mm.

As is evident from FIGS. 7A to 8B, most of light is distributed around the surface containing a straight line connecting the light irradiation unit 400 and the light detection unit 401. The biomedical light measuring apparatus 4 solves the inverse problem consistently by measuring the reflected light intensity distribution to calculate the state of light absorption (the shape, position, and optical constant of the absorber) inside the body. The reflected light intensity distribution used in this case may be set to a range containing optical paths as shown in FIGS. 7A to 8B and there is no need to include all space. By narrowing down objects of calculation to a range containing dominant data as shown above, the amount of data to be handled can be reduced and as a result, the calculation time can be shortened.

The present inventors investigated the range of space assiduously and clarified the technique of determining the range intended for calculation using each distance between the position of the light irradiation unit 400 (that is, the light irradiation position into the body) and the position of each of the light detection units 401 (that is, each detection position of emitted light from inside the body) as a reference and discriminating the optical signal used for calculation from a plurality of detected optical signals. As an example, a case when a circle whose diameter is the straight line connecting the position of the light irradiation unit 400 and the position of each of the light detection units 401 is used will be described.

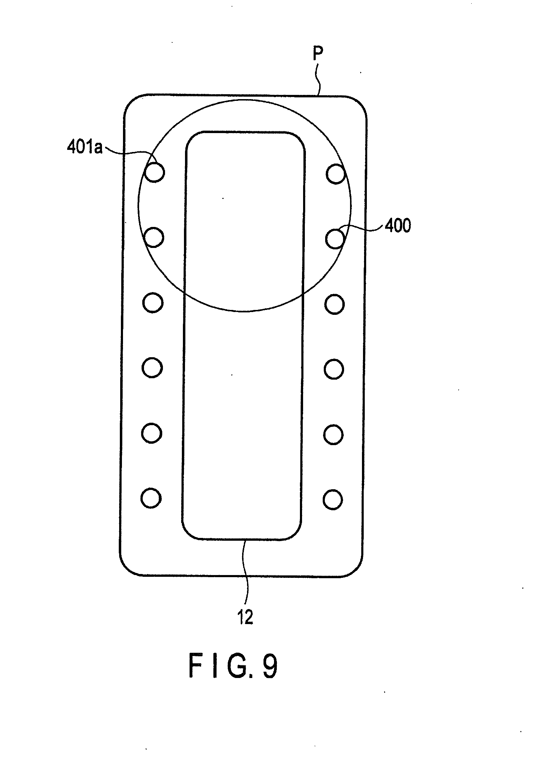

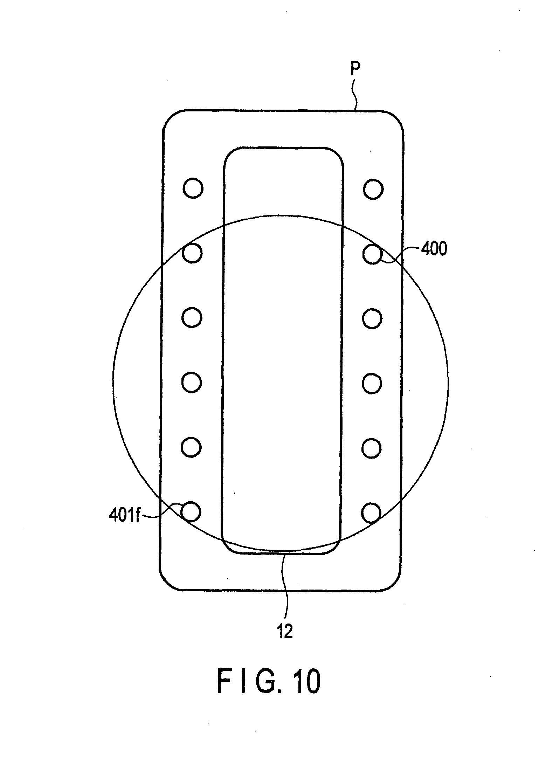

FIGS. 9, 10, and 11 are diagrams showing the contact surface PS with the body of the interface unit P and diagrams illustrating the detection signal discrimination function. In FIG. 9, a circle in which the light irradiation unit 400 and a light detection unit 401a are both ends of the diameter thereof falls within the contact surface PS. Thus, an optical signal from the body detected by the light detection unit 401a for which the detection distance is relatively short propagates through a region under the same pressing conditions and can also be said to use a region dominant for calculation as propagation paths. In FIG. 10, on the other hand, a portion of a circle in which the light irradiation unit 400 and a light detection unit 401f are both ends of the diameter thereof protrudes from the contact surface PS. Thus, an optical signal from the body detected by the light detection unit 401f for which the detection distance is relatively long does not propagate through a region under the same pressing conditions and cannot necessarily be said to use a region dominant for calculation as propagation paths. In addition, when the detection distance is long, the signal strength is low, causing an error in operation. Therefore, the optical signal detected by the light detection unit 401f does not effectively contribute to computation to calculate the state of light absorption (the shape, position, and optical constant of the absorber) inside the body and is not included for calculation.

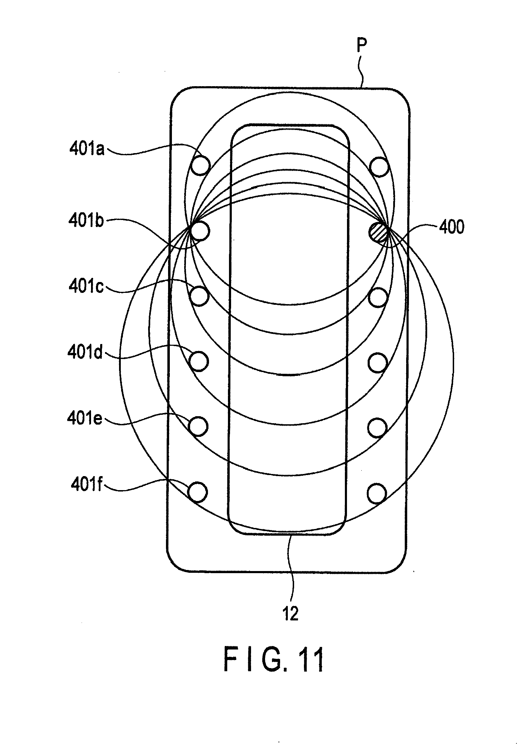

FIG. 11 shows circles in which each of light detection units 401a to 401f and the light irradiation unit 400 are both ends of the diameter thereof. As is evident from FIG. 11, the light analysis unit 424 discriminates, among optical signals detected by each of the light detection units 401a to 401f, optical signals detected by the light detection units 401a to 401c as optical signals used to calculate the state of light absorption inside the body based on the relationship between the contact surface PS and the circle in which the light irradiation unit 400 and the light detection unit 401a are both ends of the diameter thereof. Using the discriminated optical signals, the arithmetic circuit 426 calculates the state of light absorption inside the body. Alternatively, the arithmetic circuit 426 may calculate the state of light absorption inside the body by allocating a large weight coefficient to optical signals detected by the light detection units 401a to 401c and a small weight coefficient to other optical signals.

### (Operation)

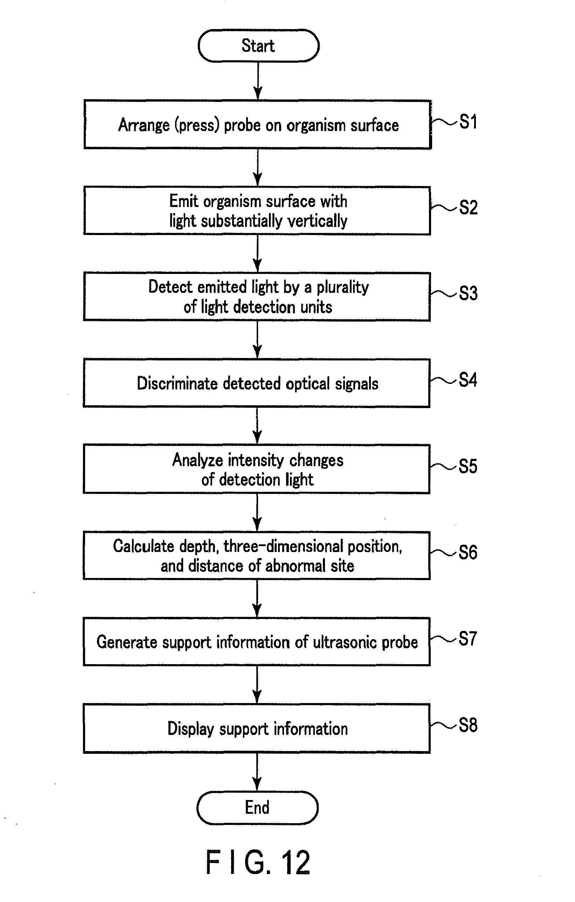

Next, guidance processing of an ultrasonic probe using the biomedical light measuring apparatus 4 according to the present embodiment will be described. FIG. 12 shows a flow chart of guidance processing of an ultrasonic probe using the ultrasonic diagnosis apparatus 1. As shown in FIG. 12, when the interface unit P is arranged on (pressed against) the body surface (step S1) and the body surface is irradiated with light substantially vertically from the light irradiation unit 400 (step S2), light diffused and reflected inside the body is detected by the plurality of light detection units 401 while being guided by a plurality of the light guiding units 402 (step S3).

The light analysis unit 424 discriminates, among optical signals detected by the plurality of light detection units 401, optical signals used to calculate the state of light absorption inside the body based on the relationship between the contact surface PS and the circle in which the light irradiation unit 400 and the light detection unit 401a are both ends of the diameter thereof (step S4). The light analysis unit 424 amplifies and then converts the discriminated optical signals (analog signals) into digital signals and analyzes changes in intensity of detection signals between the light detection units 401 (step S4).

Based on changes in intensity of detection light between discriminated optical signals, the arithmetic circuit 426 calculates the degree of contact between the ultrasonic probe 12 and the surface of the subject, the depth of an abnormal site showing a predetermined optical absorption coefficient inside the subject from the surface of the subject, and the position and distance of the abnormal site related to a predetermined position (step S6).

The support information generation unit 44 generates support information that instructs or guides the position, posture, and orientation of the ultrasonic probe 12 based on determination results and calculation results of the arithmetic circuit 426 to bring the transmitting and receiving surface of the ultrasonic probe 12 into close contact with the subject (step S7). The generated support information is displayed in the monitor 14 in a predetermined form (step S8).

### (Modification 1)

In the above embodiment, a configuration in which the range to be calculated is determined by using each distance between the light irradiation position into a body and each detection position of emitted light from inside the body as a reference and optical signals detected by each of the detection units 401 is shown. However, the present embodiment is not limited to such an example and, for example, the detection unit 401 to be used for light detection may be discriminated using each distance between the light irradiation position into the body and each detection position of emitted light from inside the body so that optical signals detected by the discriminated light detection unit 401 are used for calculation.

### (Modification 2)

The above embodiment is configured to perform processing following the detection signal discrimination function by the light analysis unit 424. However, the present embodiment is not limited to such an example and, for example, processing following the detection signal discrimination function by the arithmetic circuit 426.

According to the present embodiment described above, the range to be calculated is determined by using each distance between the light irradiation position into a body and each detection position of emitted light from inside the body as a reference, optical signals to be used for calculation are discriminated from a plurality of detected optical signals, the state of light absorption inside the body is calculated by using the discriminated optical signals. Accordingly, calculation objects can reasonably be narrowed down to a range containing dominant data so that the amount of data to be handled can be reduced. As a result, the calculation time can be made shorter and also a biomedical light measuring apparatus and an ultrasonic diagnosis apparatus that are inexpensive and highly responsive can be realized.

### (Second embodiment)

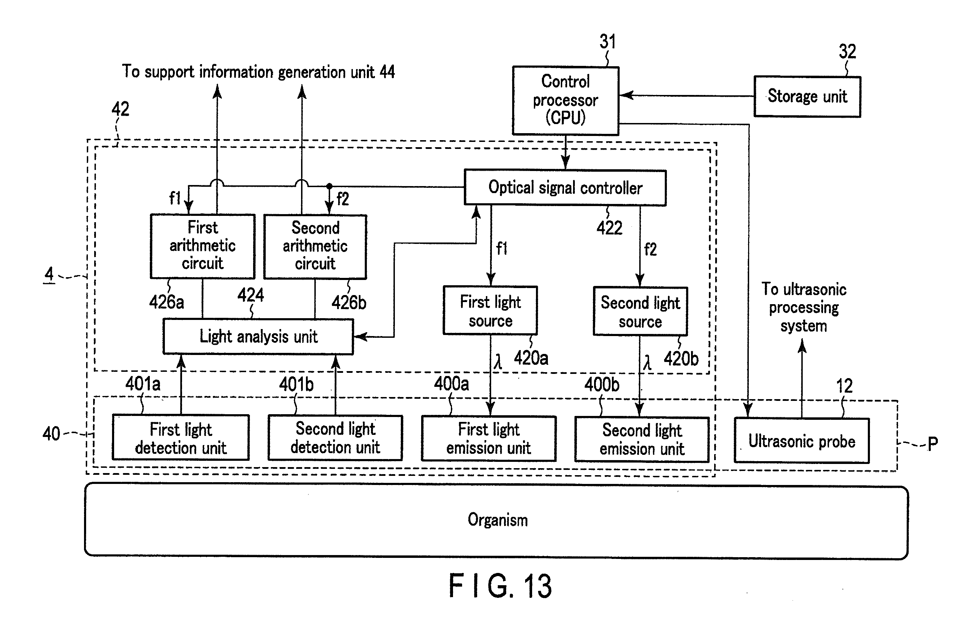

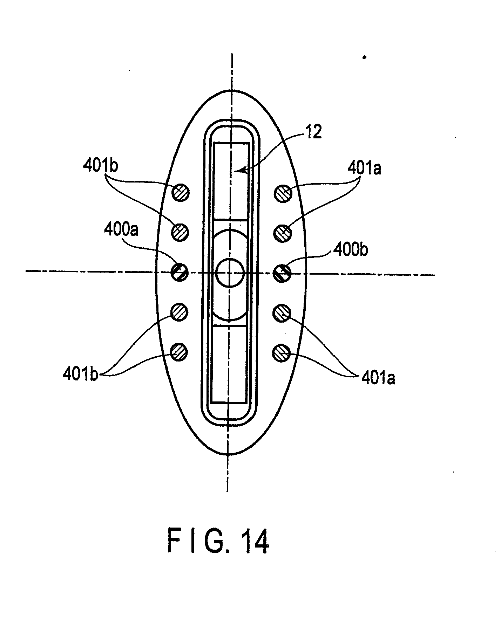

FIG. 13 is a block diagram of a biomedical light measuring apparatus 4 including a light probe 40 and an optical measurement processing unit 42. FIG. 14 is a diagram when a probe P is viewed from a subject contact surface side and is a diagram showing an arrangement example of a light irradiation unit 400 and a light detection unit 401 with respect to an ultrasonic transmitting and receiving surface of an ultrasonic probe 12.

As shown in FIGS. 13 and 14, the light probe 40 includes at least two light irradiation units 400 (in the example of FIG. 13, two units, a first light irradiation unit 400a and a second light irradiation unit 400b arranged on both sides of the center of the ultrasonic transmitting and receiving surface of the ultrasonic probe 12) and a plurality of the light detection units 401 arranged across the first light irradiation unit 400a and the second light irradiation unit 400b on both sides of the center of the ultrasonic transmitting and receiving surface of the ultrasonic probe 12. The plurality of light detection units 401 is classified into light detection units 401a belonging to the first group and light detection units 401b belonging to the second group. The light detection unit 401a belonging to the first group is allocated to the first light irradiation unit 400a in rough adjustment mode (described later) and allocated to the second light irradiation unit 400b in fine adjustment mode (described later). On the other hand, the light detection unit 401b belonging to the second group is allocated to the second light irradiation unit 400b in rough adjustment mode and allocated to the first light irradiation unit 400a in fine adjustment mode.

In FIG. 14, an example in which the plurality of light detection units 401 is classified into two groups and light detection units of each group are arranged along the longitudinal direction of the ultrasonic transmitting and receiving surface of the ultrasonic probe 12 across light incident units 400, two light detection unit on each side, is shown (in the example of FIG. 14, light incident units 400a, 400b are arranged symmetrically with respect to the longitudinal direction of the ultrasonic transmitting and receiving surface, the light detection unit 401a belonging to the first group and the light detection unit 401b belonging to the second group are arranged symmetrically with respect to the longitudinal direction of the ultrasonic transmitting and receiving surface, and the light detection units 401 belonging to the same group are arranged symmetrically with respect to the transverse direction of the ultrasonic transmitting and receiving surface). However, the present embodiment is not limited to such an example and the number of groups into which the plurality of light detection units 401 is classified may be any number equal to 2 or greater and the number of the detection units 401 in the same group may also be any number equal to 2 or greater. To maintain spatial symmetry, however, the number of groups into which the plurality of light detection units 401 is classified and the number of the detection units 401 in the same group are preferably even numbers. In the example of FIG. 14, the light incident unit 400 and the plurality of light detection units 401 are arranged at equal intervals along the longitudinal direction of the ultrasonic transmitting and receiving surface. Also, the above example is only an example and a modification that adjusts the position interval in consideration of the distance of the opposed light detection unit 401 is known.

The first light irradiation unit 400a and the second light irradiation unit 400b irradiate a subject with light (near infrared light) generated by a first light source 420a and a second light source 420b respectively. In addition, mutually different drive frequencies (f1, f2) are supplied to the first light irradiation unit 400a and the second light irradiation unit 400b. As a result, lights of specific wavelengths are irradiated from the first light irradiation unit 400a and the second light irradiation unit 400b at different drive frequencies.

The plurality of light detection units 401 has a detection surface formed from, for example, an end of an optical fiber and includes a plurality of detection elements that make a photoelectric conversion of reflected light from inside the subject input from the detection surface via the light wave-guiding unit. As the detection element, for example, in addition to a light receiving element such as a photo-diode and a photo-transistor, CCD, APD, or a photomultiplier can be adopted. An optical matching layer may be provided on the contact surface of the light irradiation unit 400 and each of the light detection units 401 with the subject.

The optical measurement processing unit 42 includes the first light source 420a, the second light source 420b, an optical signal controller 422, a light analysis unit 424, a first arithmetic circuit 426a, and a second arithmetic circuit 426b.

The first light source 420a and the second light source 420b are a light-emitting element such as a semiconductor laser, a light-emitting diode, a solid-state laser, or a gas laser that generates light of a wavelength whose absorption in a body is small (for example, light in the range of about 600 nm to 1800 nm, a wavelength range called an in vivo window) or light of a specific wavelength whose amount of absorption increases in an abnormal site (for example, light in the wavelength range called the in vivo window and also in the wavelength range of 750 to 860 nm absorbed by hemoglobin in blood). Lights generated by the first light source 420a and the second light source 420b are supplied to the first light irradiation unit 400a and the second light irradiation unit 400b via the light wave-guiding unit including the optical fiber and a thin light wave-guiding unit (or directly via propagation through the space) respectively. The first light source 420a and the second light source 420b may be configured integrally with the first light irradiation unit 400a and the second light irradiation unit 400b respectively. Different drive frequencies are supplied to the first light irradiation unit 400a and the second light irradiation unit 400b. As a result, lights of specific wavelengths are irradiated from the first light irradiation unit 400a and the second light irradiation unit 400b at different drive frequencies. In the present embodiment, it is assumed that the first light source 420a and the second light source 420b generate lights of the same wavelength. However, the present embodiment is not limited to such an example and the first light source 420a and the second light source 420b may generate lights of mutually different wavelengths.

The optical signal controller 422 controls the biomedical light measuring apparatus 4 dynamically or statically. For example, under the control of a control processor 31 of the ultrasonic diagnosis apparatus 1, the optical signal controller 422 controls the first light source 420a and the second light source 420b such that light is irradiated from the first light irradiation unit 400a and the second light irradiation unit 400b in the predetermined interval, predetermined frequency, intensity, and intensity variation period T (particularly in the present embodiment, the first light source 420a and the second light source 420b have different drive periods). The optical signal controller 422 also controls the light analysis unit 424 such that analysis processing corresponding to light of a predetermined drive period is performed in the predetermined interval.

The light analysis unit 424 includes a multi-channel lock-in amplifier and detects and amplifies a predetermined signal by selecting the period f1 or f2 and then converts the predetermined signal into a digital signal. Further, the light analysis unit 424 analyzes changes in intensity of detection signals between the light detection units 401.

The arithmetic circuit 426 calculates the degree of contact between the plurality of light detection units 401 and the surface of the subject, the depth of an abnormal site showing a predetermined optical absorption coefficient (for example, a site that absorbs more light of a specific wavelength than a normal tissue) inside the subject from the surface of the subject, and a three-dimensional position and a distance of the abnormal site relative to a predetermined position (for example, the center of the light irradiation unit 400 or an ultrasonic transmitting and receiving surface of the ultrasonic probe 12) based on changes in intensity of detection light between the light detection units 401. Calculation results by the arithmetic circuit 426 are sent to the support information generation unit 44.

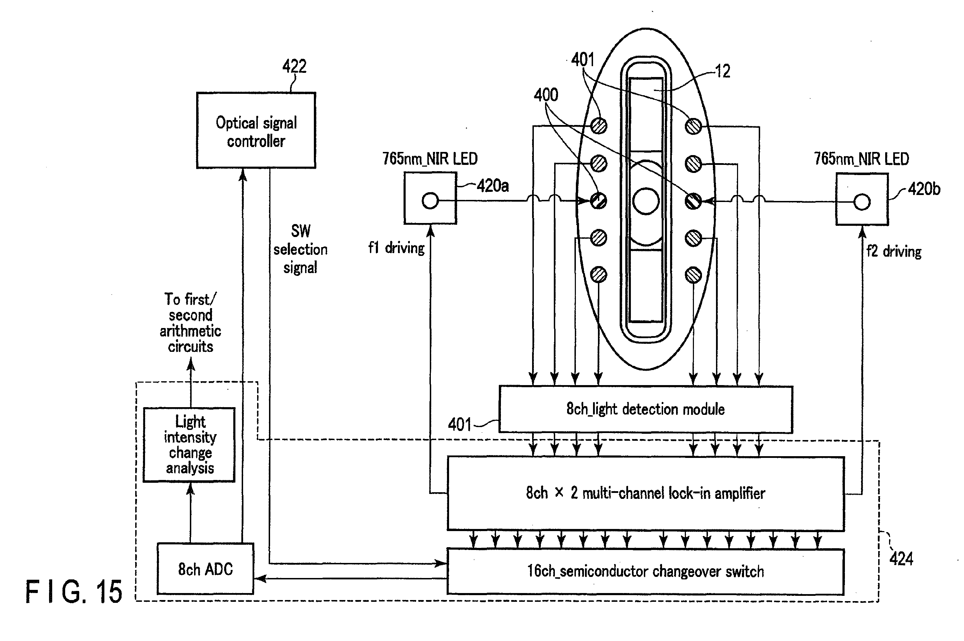

FIG. 15 is a diagram showing an example of the configuration of an optical measuring system of the biomedical light measuring apparatus 4. The optical measuring system of the biomedical light measuring apparatus 4 will be described in more detail following FIG. 15. The first light source 420a and the second light source 420b adopt, for example, LEDs whose wavelength is near 765 nm. The two LEDs are driven to blink by different drive frequencies (f1, f2). The drive frequencies (f1, f2) use a reference signal for phase detection of a multi-channel lock-in amplifier described later. Lights generated by the first light source 420a and the second light source 420b are transmitted from the LEDs to the first light irradiation unit 400a and the second light irradiation unit 400b via an optical fiber respectively. Lights are emitted from the first light irradiation unit 400a and the second light irradiation unit 400b toward the body and light from the body enters each of the light detection units 401 before being transmitted to a light detection module configured by a photoelectric conversion element (such as a photo-diode, avalanche photo-diode, and optical transistor) and a signal amplifier. An amplified light intensity signal is separated into intensity for each light source (each drive frequency) by the multi-channel lock-in amplifier held by the light analysis unit 424. In the light analysis unit 424, a 16ch signal is reduced down to an 8ch signal by a multi-channel semiconductor changeover switch and an optical signal from a selected and needed incident position is A/D converted by an AD converter to analyze intensity changes thereof.

Here, the drive frequencies (f1, f2) are set to, for example, 130 Hz and 230 Hz by avoiding integral multiples of commercial frequencies (50 Hz, 60 Hz) and considering dynamic measurement support (data update period of 10 Hz or more) and LED drive frequencies. Instead of the example shown in FIG. 15, a measuring system using all 16ch signals can also be built. However, as will be described later, if many signals are used simultaneously by combining a plurality of lights having different wavelengths, the circuit increases in size. Considering the above point, FIG. 15 illustrates a configuration in which signals are cut in half.

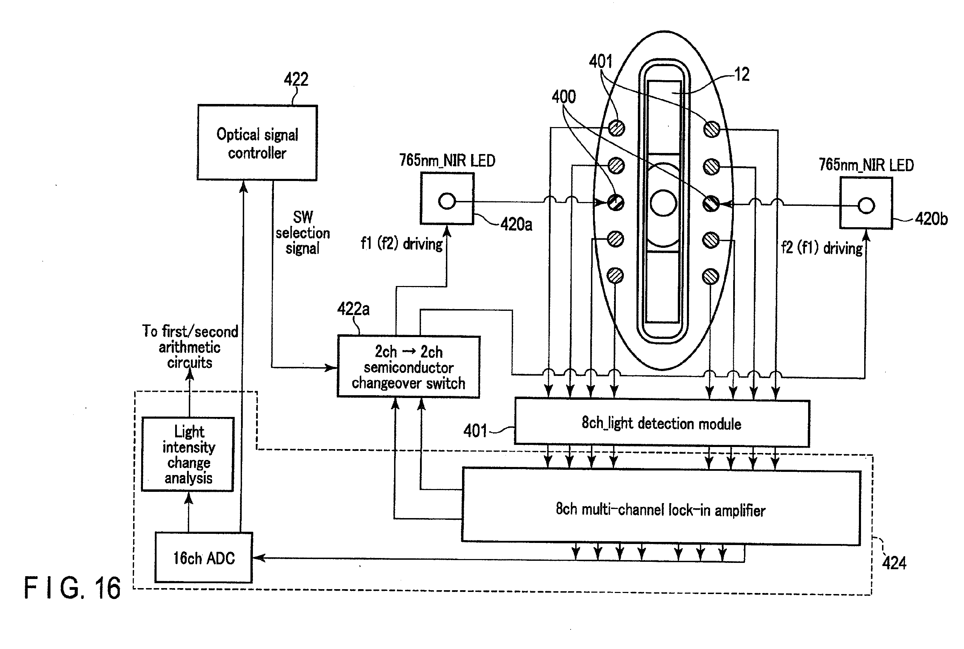

FIG. 16 is a diagram showing Modification 1 of the configuration of an optical measuring system of the biomedical light measuring apparatus 4. FIG. 16 is different from FIG. 15 in that a configuration in which instead of selecting the light source of the detection unit by the semiconductor changeover switch, the drive frequency of LED is selected by a changeover switch 422a is adopted. The number of switches can advantageously be reduced. Because the phase detection frequency for each detector is determined in advance, the system disadvantageously becomes inflexible.

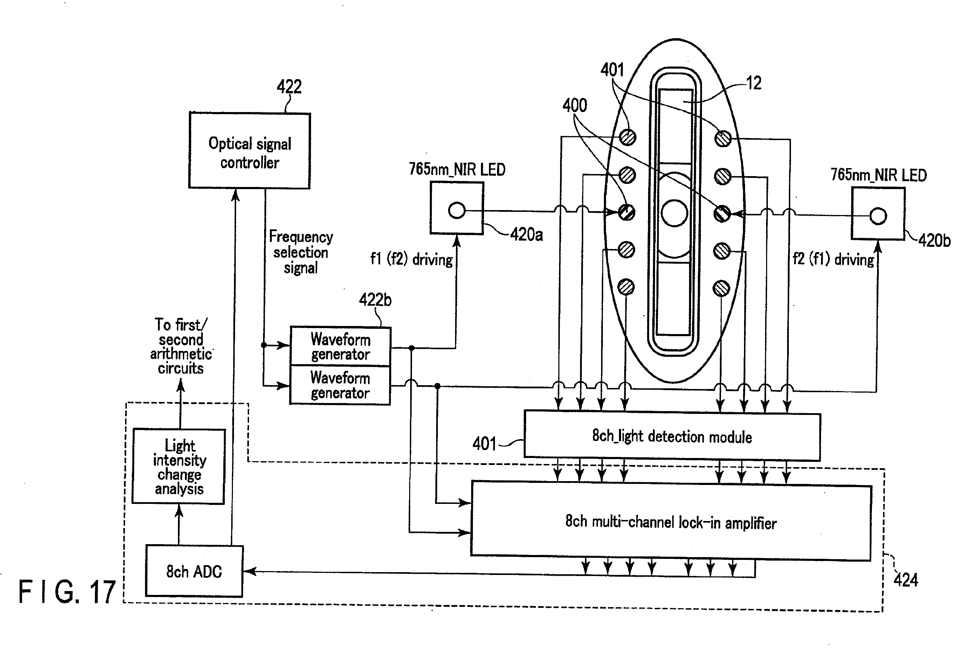

FIG. 17 is a diagram showing Modification 2 of the configuration of the optical measuring system of the biomedical light measuring apparatus 4. FIG. 17 is different from FIGS. 15 and 16 in that LED is driven by a waveform generator 422b being controlled by the optical signal controller 422 and a reference signal is sent from the waveform generator 422b to the multi-channel lock-in amplifier. In this case, the multi-channel lock-in amplifier does not need a reference signal generator. Changes of noise frequency mixed from surroundings can advantageously be handled easily. Because it is necessary to widen band-pass characteristics of a frequency filter of the lock-in amplifier, phase detection performance may disadvantageously fall.

### (Ultrasonic probe guiding function)

Next, the ultrasonic probe guiding function held by the present ultrasonic diagnosis apparatus will be described. The present function is a function that guides the position, orientation, posture, and degree of press of the ultrasonic probe 12 more suitably by irradiating the inside of a body with at least two lights irradiated at different drive frequencies, detecting an abnormal site by selecting the light detection unit corresponding to a specific drive frequency, and using analysis results of the detection.

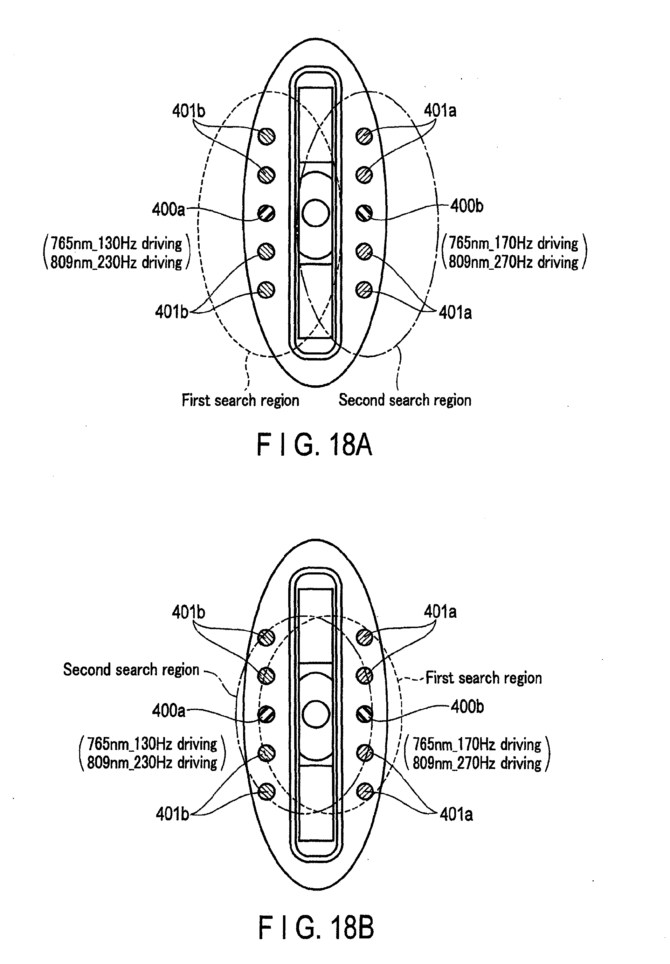

FIGS. 18A and 18B are diagrams illustrating an ultrasonic probe guiding function held by the ultrasonic diagnosis apparatus. More specifically, FIG. 18A is a diagram illustrating a mode (rough search mode) to guide the position of the ultrasonic probe 12 by performing a rough search of an abnormal site when the position of the ultrasonic probe 12 on the body surface is adjusted such that an ultrasonic scanning section by the ultrasonic probe 12 contains a site to be diagnosed (a site recognized as the abnormal site or a site suspected of abnormality) inside the body.

FIG. 18B is a diagram illustrating a mode (fine adjustment mode) to guide the position of the ultrasonic probe 12 by searching for the abnormal site with high precision so that the site to be diagnosed is contained in the ultrasonic scanning section when the position of the ultrasonic probe 12 on the body surface is similarly adjusted.

In rough search mode, the first light irradiation unit 400a and the light detection unit 401b belonging to the second group are paired and the second light irradiation unit 400b and the light detection unit 401a belonging to the first group are paired (that is, the light incident unit and the detection unit arranged on the same side of the ultrasonic probe 12 are paired).

After the pairing, biomedical light measurements are made with a region (first search region) searched by the first light irradiation unit 400a and the light detection unit 401b belonging to the second group and a region (second search region) searched by the second light irradiation unit 400b and the light detection unit 401a belonging to the first group. If, for example, a signal in a specific frequency band corresponding to a site to be diagnosed is detected more intensely in the second region than the first region, it is evident that the site to be diagnosed is present on the side of the second search region from the center (long axis) of the ultrasonic probe 12. Conversely, if the signal of the specific frequency band is detected more intensely in the first region than the second region, it is evident that the site to be diagnosed is present on the side of the first search region from the center (long axis) of the ultrasonic probe 12. In the rough search mode, regions to search for an abnormal site can be taken relatively widely on both sides of an ultrasonic irradiation surface of the ultrasonic probe 12. From the above feature, this mode can be said to be a mode suitable for guiding a site to be diagnosed up to the vicinity of the ultrasonic probe.

On the other hand, the fine adjustment mode is executed after, for example, rough position adjustments are made by undergoing the rough search mode. In fine adjustment mode, the first light irradiation unit 400a and the light detection unit 401a belonging to the first group are paired and the second light irradiation unit 400b and the light detection unit 401b belonging to the second group are paired (that is, the light incident unit and the detection unit arranged across the ultrasonic probe 12 are paired).

After the pairing, biomedical light measurements are made with a region (first search region) searched by the first light irradiation unit 400a and the light detection unit 401a belonging to the first group and a region (second search region) searched by the second light irradiation unit 400b and the light detection unit 401b belonging to the second group. At this point, for example, by comparing the intensity of a signal of a specific frequency band corresponding to a site to be diagnosed in the first search region and the intensity thereof in the second search region, the position of the site to be diagnosed can be narrowed down to the vicinity of the center of the probe. In fine adjustment mode, the region to search for an abnormal site is set so as to contain the ultrasonic irradiation surface of the ultrasonic probe 12. From the above feature, this mode can be said to be a mode suitable for fine adjustments to correctly guide the site to be diagnosed into an ultrasonic scanning plane.

In rough search mode and fine adjustment mode, information about the calculated position of a site to be diagnosed is sent to the support information generation unit 44. The support information generation unit 44 generates and outputs support information to guide the position, orientation, posture, and degree of press of the ultrasonic probe 12 more suitably with respect to the subject and the site to be diagnosed based on the received information about the site to be diagnosed. The output support information is displayed on a monitor 14 in a predetermined form.

The rough search mode and the fine adjustment mode are switched by, for example, an operation from an input apparatus 13. However, the present embodiment is not limited to such an example and, for example, the apparatus may be made to automatically select the rough search mode or the fine adjustment mode by comparing the distance between the site to be diagnosed and the ultrasonic transmitting and receiving surface and a predetermined threshold.

### (Reference wavelength light intensity calibration function)

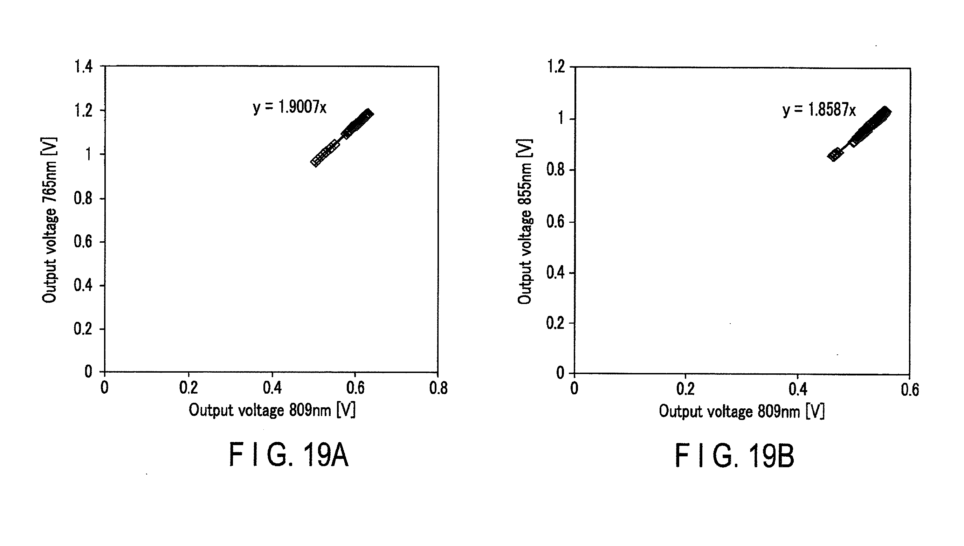

Next, the reference wavelength light intensity calibration function held by the present ultrasonic diagnosis apparatus will be described. FIG. 19A is a graph showing a correlation of light intensity between the wavelength (about 765 nm) at which deoxygenated hemoglobin is detected and a reference wavelength (about 809 nm) when measured in the same light source position and the same detection position. FIG. 19B is a graph showing the correlation of light intensity between the wavelength (about 855 nm) at which oxygenated hemoglobin is detected and the reference wavelength (about 809 nm) when similarly measured in the same light source position and the same detection position. As is evident from both graphs, regardless of the wavelength, an almost proportional relationship of correlation of light intensity is obtained.

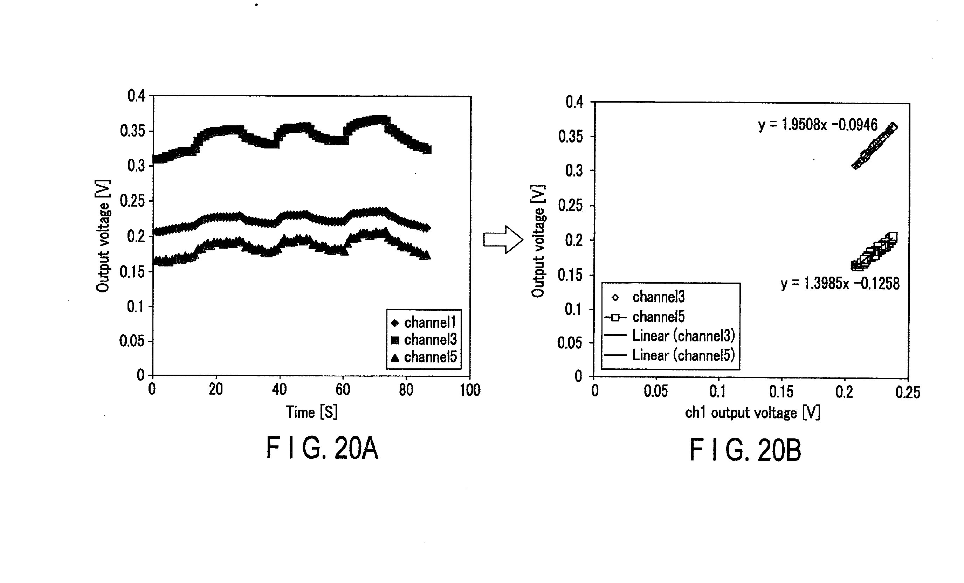

More correctly, if the distance between the light source and the detection position is made longer, an intercept arises in the straight line of correlation. For example, FIG. 20A is a graph showing changes with time of voltages output by each of the detection units 401 (ch1 = 15 mm, ch3 = 25 mm, ch5 = 35 mm) having different distances from the light source by an operation of pressing and releasing the probe P on the surface of the body. FIG. 20B is a graph showing a correlation of detected light intensity between channels normalized by intensity of the shortest distance ch1 = 15 mm. It is evident from FIGS. 20A and 20B, the intercept of the straight line of correlation increases with an increasing distance between the light source and the detection position. The reason therefor is as follows. The main factor in variations of the amount of light is a phenomenon in which blood decreases or increases by being pressed out from the vicinity of the skin due to pressurization or deformation. Thus, if the distance increases, information in deeper positions relatively increases by being reflected in the measured amount of light, which appears as an intercept of the straight line of correlation. Therefore, the selection of the reference wavelength is important and if, for example, the absorption of water (about 950 nm) is selected, an intercept appears in the straight line of correlation even in a short distance, causing an inconvenience for corrections. In the present embodiment, for example, 809 nm is adopted as the reference wavelength.

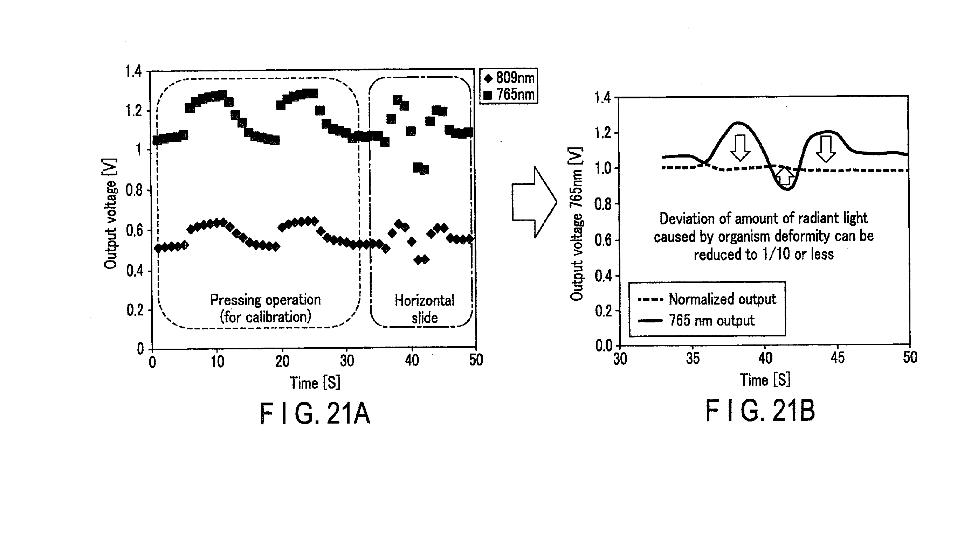

FIGS. 21A and 21B show an example of a calibration sequence that calibrates output variations in light intensity generated in biomedical light measurements based on the reference wavelength. First, as a preparation for the biomedical light measurement processing, biomedical light measurement is made while an operation of pressing the probe P against the body surface (skin) in the reference position and releasing the probe. The first arithmetic circuit 426a and the second arithmetic circuit 426b calculate a reference wavelength calibrating straight line corresponding to each incident/detection position specific to individual subjects. Then, as shown in FIG. 21A, light intensity is measured by horizontally scanning the probe P and then calibrated and normalized to form as a signal. By introducing this method, as shown in FIG. 21B, variation noise can be reduced to 1/10 or less. To make a correct evaluation taking the incident/detection distance into consideration, instead of normalization based on the reference wavelength intensity, a method of making a quantitative determination of detection data based on a deviation from a calculated calibrating straight line.

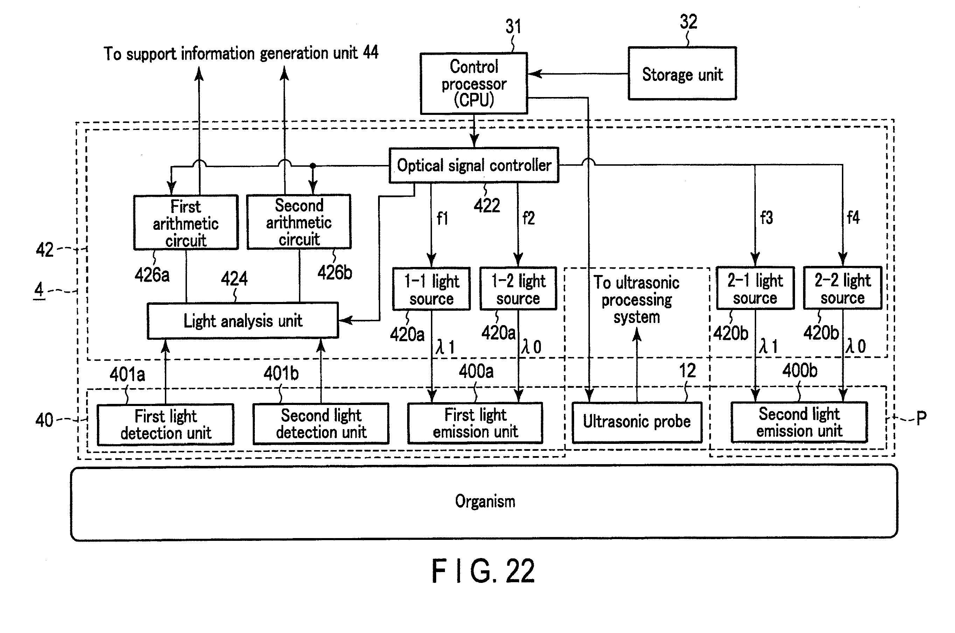

FIG. 22 is a block diagram of the biomedical light measuring apparatus 4 to which the reference wavelength light intensity calibration function is added. FIG. 22 is mainly different from the configuration in FIG. 13 in that lights are guided from two light sources (wavelength: λ1, λ2) to each of the first light irradiation unit 400a and the second light irradiation unit 400b and a 1-1 light source 420a, a 1-2 light source 420a, a 2-1 light source 420b, and a 2-2 light source 420b are all driven by separate frequencies (f1, f2, f3, and f4).

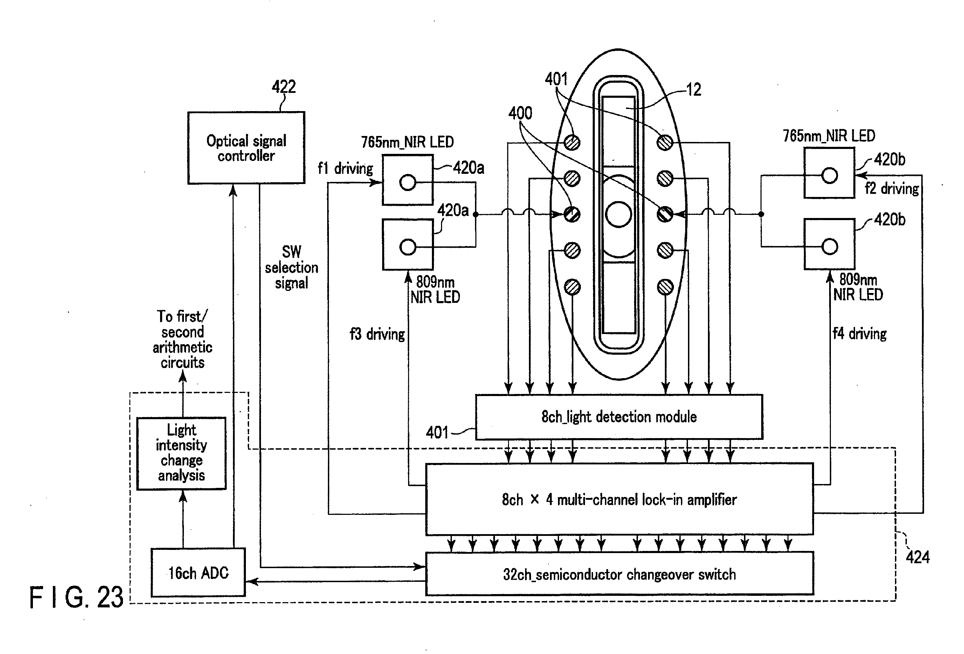

FIG. 23 is an example of the configuration of the optical measuring system of the biomedical light measuring apparatus 4 to which the reference wavelength light intensity calibration is added. FIG. 23 is different from FIG. 15 in that lights are guided from two light sources (wavelength: λ1, λ2. λ1 = 765 nm and λ2 = 809 nm in the example of FIG. 23) to each of the first light irradiation unit 400a and the second light irradiation unit 400b, the 1-1 light source 420a, the 1-2 light source 420a, the 2-1 light source 420b, and the 2-2 light source 420b are all driven by different frequencies (f1, f2, f3, and f4), a drive signal of LED is supplied from the multi-channel lock-in amplifier like in FIG. 15, but has four frequencies, and the multi-channel lock-in amplifier is doubly as large as in FIG. 15 with 8ch x 4 configuration and has a multi-channel semiconductor changeover switch that reduces a 32ch signal down to a 16ch signal.

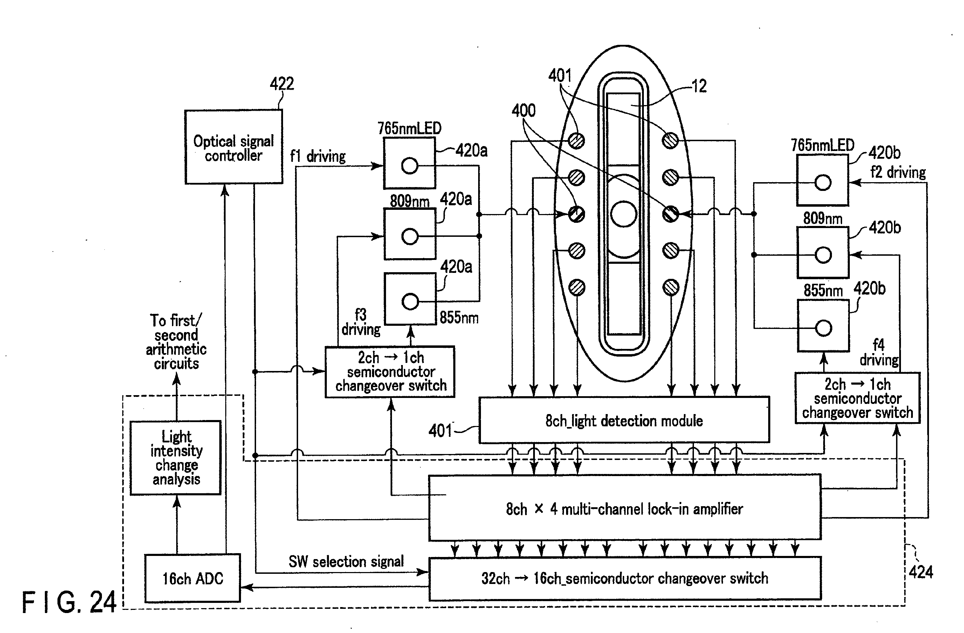

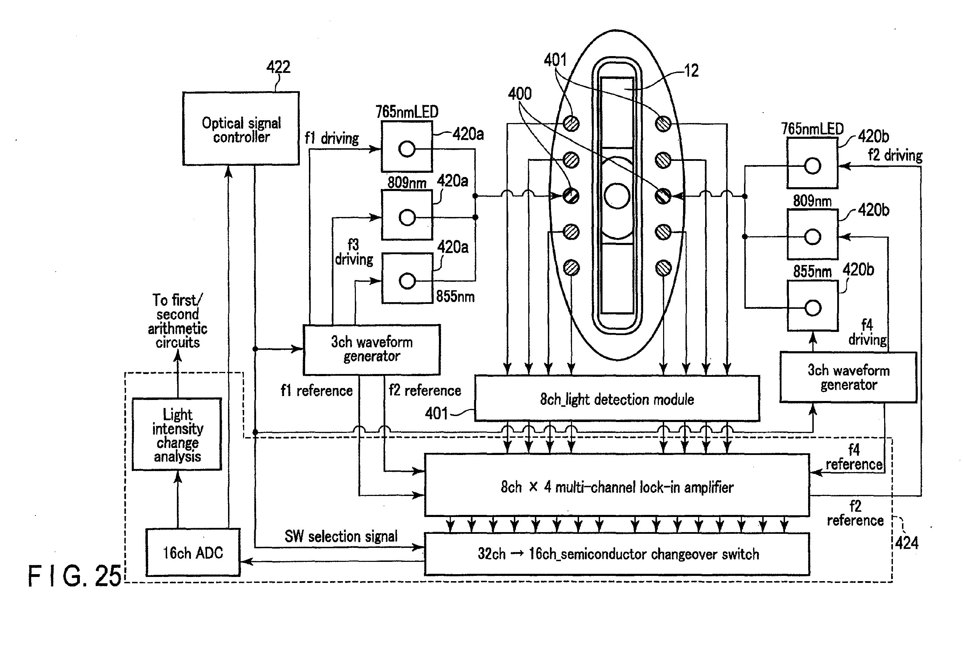

FIG. 24 is an example of the configuration of the optical measuring system of the biomedical light measuring apparatus 4 to which the reference wavelength light intensity calibration and the precise measuring function of necessary for visualization of images of light absorption is added. FIG. 25 is different from FIG. 23 in that lights are guided from three light sources (wavelength: λ1, λ2, λ3. λ1 = 765 nm, λ2 = 809 nm, and λ3 = 855 nm in the example of FIG. 24) to each of the first light irradiation unit 400a and the second light irradiation unit 400b and a semiconductor changeover switch to select two pairs (four) of LED driven simultaneously from three pairs (six) is arranged. Naturally, a configuration that simultaneously drives three pairs of LED can be considered, but in this example, the selection mechanism is provided. The system circuit size is reduced by separately using 765 nm and 809 nm for guidance and 765 nm and 855 nm for visualization calculation of light absorption.

FIG. 25 is another example of the configuration of the optical measuring system of the biomedical light measuring apparatus 4 to which the reference wavelength light intensity calibration and the precise measuring function is added. FIG. 25 is different from FIG. 24 in that LED is driven like in FIG. 17 by driving the waveform generator by the optical signal controller 422 and a reference signal is sent from the waveform generator to the multi-channel lock-in amplifier.

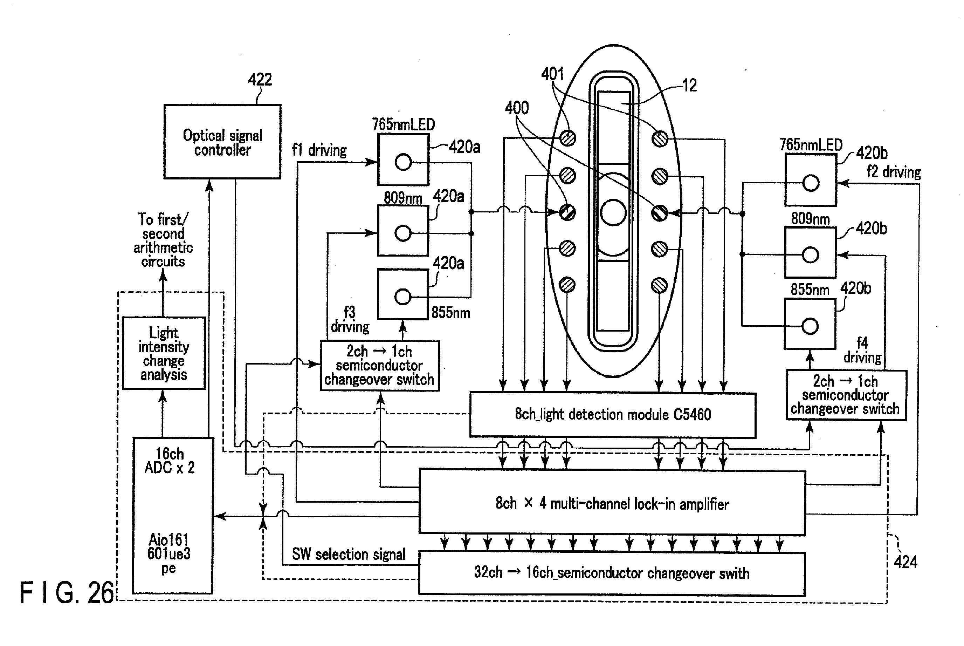

FIG. 26 is another example of the configuration of the optical measuring system of the biomedical light measuring apparatus 4 to which the reference wavelength light intensity calibration is added. In this system, an approximate mode in which signal processing in 16ch is performed at high speed using an analog divider and a precise mode in which all 32ch signals are fetched and analyzed by signal processing as software can separately be used.

According to an ultrasonic diagnosis apparatus in the present embodiment described above, the inside of a body is irradiated with at least two lights irradiated at different drive frequencies to be able to detect an abnormal sire while selecting the light detection unit corresponding to a specific drive frequency. Thus, the object to be measured can be guided to immediately below the center position of the ultrasonic probe 12 by excluding interference of lights from a plurality of light sources. Particularly in the present ultrasonic diagnosis apparatus, a site to be diagnosed can be guided to the vicinity of the ultrasonic probe in rough search mode and the site to be diagnosed can be arranged with high precision in the ultrasonic scanning plane by guiding the site easily and swiftly in fine adjustment mode.

Also according to the present ultrasonic diagnosis apparatus, an operation to press the probe P against the body surface and releasing the probe in the reference position using the reference wavelength light intensity calibration function to calculate a reference wavelength calibrating straight line corresponding to each incident/detection position specific to individual subjects and light intensity is measured by horizontally scanning the ultrasonic probe and then calibrated and normalized, which can then be formed as a signal. Accordingly, variation noise caused by the operation to press the probe P against the body surface and releasing the probe can dramatically be reduced.

In the embodiments described above, the biomedical light measuring apparatus 4 and the ultrasonic diagnosis apparatus 1 have been described by taking a case in which both apparatuses are integrated as an example. However, the above embodiments are not limited to such an example and an ultrasonic diagnosis apparatus according to the above embodiments may be realized by mounting the light probe 40 on the ultrasonic probe 12 using an adapter and mounting the separate biomedical light measuring apparatus 4 (or hardware and a program to realize the function of the biomedical light measuring apparatus 4) on the existing ultrasonic diagnosis apparatus 1 retrospectively.

### (Third embodiment)

A biomedical light measuring apparatus according to the first embodiment and a biomedical light measuring apparatus according to the second embodiment can also be combined. In this case, the light analysis unit 424 as shown in FIG. 13 has functions of the light analysis unit 424 as shown in FIG. 1. The first arithmetic circuit 426a and the second arithmetic circuit 426b as shown in FIG. 13 have functions of arithmetic circuit 426 as shown in FIG. 1. In addition, light is emitted at different drive frequencies and the detected light is discriminated at each frequency.

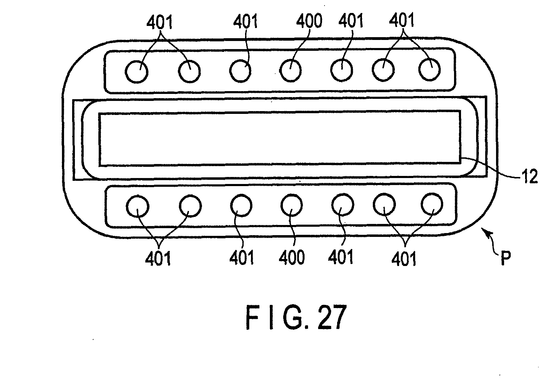

FIG. 27 is a diagram when an interface unit P according to a third embodiment is viewed from the subject contact surface side. The interface unit P shown in FIG. 27 includes functions of both of the interface unit P shown in FIG. 3 and the interface unit P shown in FIG. 14. The shape thereof is a blended shape of the rectangular shape of the interface unit P shown in FIG. 3 and the elliptical shape of the interface unit P shown in FIG. 14. However, the shape thereof is not particularly limited and the shape of the interface unit P shown in FIG. 27 can be applied to a biomedical light measuring apparatus according to the first embodiment or the second embodiment.

In FIGS. 3, 14, and 27, the light irradiation unit is arranged on both sides of the center of the long side of the ultrasonic transmitting and receiving surface. However, the arrangement position of the light irradiation unit is not particularly limited and the light irradiation unit may be arranged, for example, near one short side of the ultrasonic transmitting and receiving surface or unevenly from the center of the long side of the ultrasonic transmitting and receiving surface. In addition, light irradiation units arranged near the center of the long side of the ultrasonic transmitting and receiving surface and those arranged away from the center may be mixed.

Some embodiments of the present invention have been described, but these embodiments are presented by way of example and do not intend to limit the scope of the invention. These novel embodiments can also be carried out in other various ways and various omissions, substitutions, and alterations can be made without deviating from the spirit of the invention. These embodiments and modifications thereof are included in the scope and spirit of the invention and also included in inventions described in claims and also in equivalents thereof.

## Claims

1. An ultrasonic diagnosis apparatus **characterized by** comprising:
an ultrasonic probe that transmits an ultrasonic wave to a subject and receives the ultrasonic wave reflected inside the subject, via the ultrasonic transmitting and receiving surface;
an image generation unit configured to generate an ultrasonic image using the ultrasonic wave received by the ultrasonic probe;
a plurality of light emission units configured to be arranged around the ultrasonic transmitting and receiving surface and emit light including a component of a specific wavelength to the subject, the particular wavelength being included in an absorption wavelength range of a body;
a control unit configured to drive a plurality of the light emission units at different frequencies
a plurality of light detection units configured to be arranged around the ultrasonic transmitting and receiving surface and detect the light of the specified wavelength reflected inside the subject; and
a calculation unit configured to calculate intensity of the light of the specified wavelength at different frequencies.

2. The ultrasonic diagnosis apparatus according to claim 1, **characterized by** further comprising: an information generation unit configured to generate guidance information to guide an arrangement position on the surface of the subject of the ultrasonic probe based on the changes in intensity of the light.

3. The ultrasonic diagnosis apparatus according to claim 1, **characterized in that** a plurality of the light emission units emits the lights of a first wavelength light intensity modulated at different frequencies and corresponding to a plurality of components of the specific wavelength and a second wavelength that is different from the first component.

4. The ultrasonic diagnosis apparatus according to claim 1, **characterized in that** a plurality of the light emission units emits near infrared rays of a wavelength of at least near 809 nm.

5. The ultrasonic diagnosis apparatus according to claim 1, **characterized in that** the calculation unit normalizes intensity of first light emitted by the light emission units using intensity of second light that is different from the first light.

6. The ultrasonic diagnosis apparatus according to claim 5, **characterized in that** the calculation unit determines quantitatively intensity of the light of the specific wavelength detected by the light detection units based on a deviation from calibration information calculated by using the light of the first wavelength.

7. An ultrasonic diagnosis apparatus **characterized by** comprising:
an ultrasonic probe that transmits an ultrasonic wave to a subject and receives the ultrasonic wave reflected inside the subject, via the ultrasonic transmitting and receiving surface;
an image generation configured to generate an ultrasonic image using the ultrasonic wave received by the ultrasonic probe;
at least one light emission unit configured to be arranged around the ultrasonic transmitting and receiving surface and emit light substantially perpendicular to a surface of the subject;
a plurality of light detection units configured to be arranged around the ultrasonic transmitting and receiving surface and detect the light of the specified wavelength reflected inside the subject;
a housing having a contact surface with the subject including the ultrasonic transmitting and receiving surface, an emission surface of the at least one of the light emission units, and a detection surface of the light detection units;
a discrimination unit configured to discriminate light detected by the light detection units based on distances between the at least one of the light emission units and the light detection units;
a calculation unit that calculates a position and a size of an abnormal site showing a predetermined optical absorption coefficient inside the subject; and
a display unit that displays the position and the size of the abnormal site.

8. The ultrasonic diagnosis apparatus according to claim 7, **characterized in that** the discrimination unit discriminates the light based on a configuration relationship between circles and the contact surface, the circles having diameters which are distances between a position of the at least one of the light emission unit and a position of each of the light detection units.

9. The ultrasonic diagnosis apparatus according to claim 7, **characterized in that** when the housing is pressed in a direction substantially perpendicular to the surface of the subject, the emission surface of the at least one of the light emission units and the detection surface of the light detection units come into contact with the subject with an equal pressure.

10. The ultrasonic diagnosis apparatus according to claim 7, **characterized in that** the ultrasonic transmitting and receiving surface is substantially rectangular and
the detection units are arranged in a region sandwiched between two straight lines obtained by extending short sides of the ultrasonic transmitting and receiving surface.

11. The ultrasonic diagnosis apparatus according to claim 7, **characterized in that** the ultrasonic transmitting and receiving surface is substantially rectangular and
the detection units are arranged substantially in parallel with a long side of the ultrasonic transmitting and receiving surface.

12. The ultrasonic diagnosis apparatus according to claim 7, **characterized in that** a wavelength of the light irradiated by the light emission unit is 600 nm to 1800 nm.

13. The ultrasonic diagnosis apparatus according to claim 7, **characterized in that** at least one pair of the light emission unit and the irradiation surface on the contact surface and each of the light detection units and the detection surface on the contact surface is optically connected via an optical fiber.

14. A biomedical light measuring apparatus, which is arranged in an ultrasonic diagnostic apparatus, **characterized by** comprising:
a plurality of light emission units configured to be arranged around an ultrasonic transmitting and receiving surface of an ultrasonic probe included by the ultrasonic diagnostic apparatus and emit light including a component of a specific wavelength to the subject, the particular wavelength being included in an absorption wavelength range of a body;
a control unit configured to drive a plurality of the light emission units at different frequencies
a plurality of light detection units configured to be arranged around the ultrasonic transmitting and receiving surface and detect the light of the specified wavelength reflected inside the subject; and
a calculation unit configured to calculate intensity of the light of the specified wavelength at different frequencies.

15. A biomedical light measuring apparatus, which is arranged in an ultrasonic diagnostic apparatus, **characterized by** comprising:
at least one light emission unit configured to be arranged around an ultrasonic transmitting and receiving surface of an ultrasonic probe included by the ultrasonic diagnostic apparatus and emit light substantially perpendicular to a surface of the subject;
a plurality of light detection units configured to be arranged around the ultrasonic transmitting and receiving surface and detect the light of the specified wavelength reflected inside the subject;
a housing having a contact surface with the subject including the ultrasonic transmitting and receiving surface, an emission surface of the at least one of the light emission units, and a detection surface of the light detection units;
a discrimination unit configured to discriminate light detected by the light detection units based on distances between the at least one of the light emission units and the light detection units;
a calculation unit that calculates a position and a size of an abnormal site showing a predetermined optical absorption coefficient inside the subject; and
a display unit that displays the position and the size of the abnormal site.
